# EUROPEAN PATENT APPLICATION

(11) **EP 2 088 192 A1**
(43) Date of publication of application: **12.08.2009**
(21) Application number: 07830247.8
(22) Date of filing: 16.10.2007
(51) Int. Cl.: C12N 9/99, C12N 1/04, C12N 1/06, C12N 1/21, C12N 15/09, C12P 21/00, C07K 14/32

(54) **LYTIC ENZYME INHIBITOR, LYSIS INHIBITOR, INHIBITOR OF DEGRADATION OF POLY- GAMMA-GLUTAMIC ACID, AND METHOD FOR PRODUCTION OF POLY- GAMMA-GLUTAMIC ACID**

(30) Priority: 16.10.2006 JP 2006281768
(71) Applicant: Kao Corporation, Chuo-Ku Tokyo 103-8210 (JP); Shinshu University, Matsumoto-shi, Nagano 390-8621 (JP)
(72) Inventor: ARA, Katsutoshi, Haga-gun Tochigi 321-3497 (JP); SEKIGUCHI, Junichi, Ueda-shi Nagano 386-8567 (JP); YAMAMOTO, Hiroki, Ueda-shi Nagano 386-8567 (JP); HARADA, Hiroyuki, Ueda-shi Nagano 386-8567 (JP)
(74) Representative: Vossius & Partner
(86) International application number: PCT/JP2007/070513
(87) International publication number: WO 2008/047936

(57) **Abstract**

This invention relates to inhibition of microbial lysis via regulation of activity of a lytic enzyme for degrading the cell wall. The lytic enzyme inhibitor and the lysis inhibitor are each composed mainly of the Bacillus subtilis-derived YoeB gene or the YoeB protein encoded by a gene homologous to the YoeB gene. An example of the YoeB protein is a protein consisting of the amino acid sequence as shown in SEQ ID NO: 2.

SEQUENCE LISTING

<110> KAO Corporation
      Shinshu University

<120> Lytic enzyme inhibitor, lysis inhibitor, inhibitor of poly-γ-glutamic
      acid degradation, and method for producing poly-γ-glutamic acid

<130> R1712 EP S3

<140> EP 07 83 0247.8
<141> 2007-10-16

<150> JP 2006-281768
<151> 2006-10-16

<160> 69

<170> PatentIn version 3.3

<210> 1
<211> 546
<212> DNA
<213> Bacillus subtilis

<220>
<221> CDS
<222> (1)..(546)

<400> 1
atg aaa aaa tgt ctt cta ttt cta aca acc att gca ctt att ctg tca      48
Met Lys Lys Cys Leu Leu Phe Leu Thr Thr Ile Ala Leu Ile Leu Ser
1               5                   10                  15

tta agc aca aat gca ttt gcg aaa aat aca tca ggc gat tta tca caa      96
Leu Ser Thr Asn Ala Phe Ala Lys Asn Thr Ser Gly Asp Leu Ser Gln
            20                  25                  30

aaa caa gca tta cag ttg gca tta tcc gct aga gaa cat ttt tgg aac      144
Lys Gln Ala Leu Gln Leu Ala Leu Ser Ala Arg Glu His Phe Trp Asn
        35                  40                  45

acc atg agc ggc cac aat ccg aag gtg aaa aaa gca gtt tgc cca tca      192
Thr Met Ser Gly His Asn Pro Lys Val Lys Lys Ala Val Cys Pro Ser
    50                  55                  60

ggc aca ttc gag tat caa aat ctt caa tat gta tac atg tgc agt gat      240
Gly Thr Phe Glu Tyr Gln Asn Leu Gln Tyr Val Tyr Met Cys Ser Asp
65                  70                  75                  80

cta ggc act aaa gca aaa gcg gtg aat tat tta act cct att ttt aca      288
Leu Gly Thr Lys Ala Lys Ala Val Asn Tyr Leu Thr Pro Ile Phe Thr
                85                  90                  95

aaa aca gca atc gaa aaa ggc ttt aaa gat tat cat ttc act gtt tca      336
Lys Thr Ala Ile Glu Lys Gly Phe Lys Asp Tyr His Phe Thr Val Ser
            100                 105                 110

aaa gga aaa ctt gct gtt cca atc ggt gac gga gac aat ctt ttg aat      384

Lys Gly Lys Leu Ala Val Pro Ile Gly Asp Gly Asp Asn Leu Leu Asn
        115                 120                 125

tgg aaa aaa tca act gct aaa ctg atc tct aaa aaa ggc agc aca atc        432
Trp Lys Lys Ser Thr Ala Lys Leu Ile Ser Lys Lys Gly Ser Thr Ile
    130                 135                 140

aca tac gaa ttt act gtc cct aca tta gac gga tcc cct tct gca aaa        480
Thr Tyr Glu Phe Thr Val Pro Thr Leu Asp Gly Ser Pro Ser Ala Lys
145                 150                 155                 160

cgg aaa gtg aca ttt gtg aaa gaa aat aaa aaa tgg aaa gtc aat caa        528
Arg Lys Val Thr Phe Val Lys Glu Asn Lys Lys Trp Lys Val Asn Gln
                165                 170                 175

ttt gac gca gtt ata taa                                                546
Phe Asp Ala Val Ile
            180


<210>  2
<211>  181
<212>  PRT
<213>  Bacillus subtilis

<400>  2

Met Lys Lys Cys Leu Leu Phe Leu Thr Thr Ile Ala Leu Ile Leu Ser
1               5                   10                  15


Leu Ser Thr Asn Ala Phe Ala Lys Asn Thr Ser Gly Asp Leu Ser Gln
            20                  25                  30


Lys Gln Ala Leu Gln Leu Ala Leu Ser Ala Arg Glu His Phe Trp Asn
        35                  40                  45


Thr Met Ser Gly His Asn Pro Lys Val Lys Lys Ala Val Cys Pro Ser
    50                  55                  60


Gly Thr Phe Glu Tyr Gln Asn Leu Gln Tyr Val Tyr Met Cys Ser Asp
65                  70                  75                  80


Leu Gly Thr Lys Ala Lys Ala Val Asn Tyr Leu Thr Pro Ile Phe Thr
                85                  90                  95


Lys Thr Ala Ile Glu Lys Gly Phe Lys Asp Tyr His Phe Thr Val Ser
            100                 105                 110


Lys Gly Lys Leu Ala Val Pro Ile Gly Asp Gly Asp Asn Leu Leu Asn
        115                 120                 125

Trp Lys Lys Ser Thr Ala Lys Leu Ile Ser Lys Lys Gly Ser Thr Ile
    130                 135                 140

Thr Tyr Glu Phe Thr Val Pro Thr Leu Asp Gly Ser Pro Ser Ala Lys
145                 150                 155                 160

Arg Lys Val Thr Phe Val Lys Glu Asn Lys Lys Trp Lys Val Asn Gln
                165                 170                 175

Phe Asp Ala Val Ile
            180

<210>  3
<211>  1467
<212>  DNA
<213>  Bacillus subtilis

<220>
<221>  CDS
<222>  (1)..(1467)

<400>  3
atg aaa aag aaa tta gca gca ggg ctg aca gca tct gcg att gtc ggc        48
Met Lys Lys Lys Leu Ala Ala Gly Leu Thr Ala Ser Ala Ile Val Gly
1               5                   10                  15

aca act tta gta gtg aca cca gct gaa gca gca acg att aag gtc aaa        96
Thr Thr Leu Val Val Thr Pro Ala Glu Ala Ala Thr Ile Lys Val Lys
            20                  25                  30

agc gga gat tct tta tgg aaa ctg gcc caa acc tat aac aca agt gtg       144
Ser Gly Asp Ser Leu Trp Lys Leu Ala Gln Thr Tyr Asn Thr Ser Val
        35                  40                  45

gcg gcc ctc aca tcc gcc aat cat ctt tcg act act gtt ctg tcg atc       192
Ala Ala Leu Thr Ser Ala Asn His Leu Ser Thr Thr Val Leu Ser Ile
    50                  55                  60

ggc cag acg ctt act att cca ggc agc aaa tcc agc act tct tca tct       240
Gly Gln Thr Leu Thr Ile Pro Gly Ser Lys Ser Ser Thr Ser Ser Ser
65                  70                  75                  80

act tca agc tca aca acc aag aag agc gga agt tct gtt tac aca gtg       288
Thr Ser Ser Ser Thr Thr Lys Lys Ser Gly Ser Ser Val Tyr Thr Val
                85                  90                  95

aaa agc gga gat tca ctt tgg ctg atc gca aat gag ttt aaa atg acg       336
Lys Ser Gly Asp Ser Leu Trp Leu Ile Ala Asn Glu Phe Lys Met Thr
            100                 105                 110

gtg cag gaa ctg aaa aaa tta aac gga cta agc agc gat tta att cgt       384
Val Gln Glu Leu Lys Lys Leu Asn Gly Leu Ser Ser Asp Leu Ile Arg
        115                 120                 125

gct ggg cag aaa tta aag gtg tcc ggt act gtt tct tca agc tct agt 432
Ala Gly Gln Lys Leu Lys Val Ser Gly Thr Val Ser Ser Ser Ser Ser
    130                 135                 140

tcc agt aaa aaa agc aac tct aat aag agt tca agc tcc agt tca aaa 480
Ser Ser Lys Lys Ser Asn Ser Asn Lys Ser Ser Ser Ser Ser Ser Lys
145                 150                 155                 160

agc agt tct aat aag agt tca agt tct tca tct tct acg gga aca tat 528
Ser Ser Ser Asn Lys Ser Ser Ser Ser Ser Ser Ser Thr Gly Thr Tyr
                165                 170                 175

aag gtg cag ctt gga gat tca ctt tgg aaa att gca aac aaa gtc aat 576
Lys Val Gln Leu Gly Asp Ser Leu Trp Lys Ile Ala Asn Lys Val Asn
            180                 185                 190

atg tcc atc gct gaa ttg aag gtc ttg aac aac tta aaa tca gac acc 624
Met Ser Ile Ala Glu Leu Lys Val Leu Asn Asn Leu Lys Ser Asp Thr
        195                 200                 205

att tac gtg aat cag gtg ttg aaa aca aaa tca agc ggc tct gat acg 672
Ile Tyr Val Asn Gln Val Leu Lys Thr Lys Ser Ser Gly Ser Asp Thr
    210                 215                 220

tct tct aaa gac aat tca tcc aaa tcg aac caa act tca gca aca act 720
Ser Ser Lys Asp Asn Ser Ser Lys Ser Asn Gln Thr Ser Ala Thr Thr
225                 230                 235                 240

aaa tat acc gtt aaa agc ggt gat tca ctt tgg aaa atc gca aac aac 768
Lys Tyr Thr Val Lys Ser Gly Asp Ser Leu Trp Lys Ile Ala Asn Asn
                245                 250                 255

tat aac ctg act gta cag caa atc cga aat atc aac aat ctg aaa tca 816
Tyr Asn Leu Thr Val Gln Gln Ile Arg Asn Ile Asn Asn Leu Lys Ser
            260                 265                 270

gat gtg ctt tac gtt gga caa gta ttg aag ctg aca ggt aaa gca tct 864
Asp Val Leu Tyr Val Gly Gln Val Leu Lys Leu Thr Gly Lys Ala Ser
        275                 280                 285

tca ggc tct tca tca tcg tcg tct tct tcg tca aat gca agc tcc ggc 912
Ser Gly Ser Ser Ser Ser Ser Ser Ser Ser Ser Asn Ala Ser Ser Gly
    290                 295                 300

acg acg act aca tac acg gtg aaa agc ggt gat tcc tta tgg gtg att 960
Thr Thr Thr Thr Tyr Thr Val Lys Ser Gly Asp Ser Leu Trp Val Ile
305                 310                 315                 320

gct caa aaa ttt aat gta aca gct cag cag att cgt gag aaa aac aat 1008
Ala Gln Lys Phe Asn Val Thr Ala Gln Gln Ile Arg Glu Lys Asn Asn
                325                 330                 335

tta aaa acg gat gtc ttg cag gtt ggc caa aag ctg gtc att tcc gga 1056
Leu Lys Thr Asp Val Leu Gln Val Gly Gln Lys Leu Val Ile Ser Gly
            340                 345                 350

aaa gct tca tca tcg tct tca tcc ggt tct tca aac acc acc agc tca 1104
Lys Ala Ser Ser Ser Ser Ser Ser Gly Ser Ser Asn Thr Thr Ser Ser
        355                 360                 365

acg agt gcg aag att aac aca atg att tcg gcc gct aaa gcg cag ctt      1152
Thr Ser Ala Lys Ile Asn Thr Met Ile Ser Ala Ala Lys Ala Gln Leu
    370                 375                 380

ggg gtt ccg tat cgc tgg gga ggc acg aca ccg tcc ggg ttt gac tgc      1200
Gly Val Pro Tyr Arg Trp Gly Gly Thr Thr Pro Ser Gly Phe Asp Cys
385                 390                 395                 400

agc gga ttc att tac tat gta ctg aac aaa gtc aca tcc gta tct aga      1248
Ser Gly Phe Ile Tyr Tyr Val Leu Asn Lys Val Thr Ser Val Ser Arg
                405                 410                 415

tta acg gca gcg gga tat tgg aac aca atg aaa tca gtt agc cag cct      1296
Leu Thr Ala Ala Gly Tyr Trp Asn Thr Met Lys Ser Val Ser Gln Pro
            420                 425                 430

gct gtc gga gat ttc gtt ttc ttt tca aca tat aaa gcc ggc cct tcc      1344
Ala Val Gly Asp Phe Val Phe Phe Ser Thr Tyr Lys Ala Gly Pro Ser
        435                 440                 445

cac gtc gga att tac cta ggc aac ggg gaa ttc ata aat gcc aac gat      1392
His Val Gly Ile Tyr Leu Gly Asn Gly Glu Phe Ile Asn Ala Asn Asp
    450                 455                 460

tcc gga gtt gtc att tcc aat atg aac aac tct tac tgg aaa cag cgc      1440
Ser Gly Val Val Ile Ser Asn Met Asn Asn Ser Tyr Trp Lys Gln Arg
465                 470                 475                 480

tat ctc ggt gca aaa cga tat ttc taa                                  1467
Tyr Leu Gly Ala Lys Arg Tyr Phe
                485

<210>  4
<211>  488
<212>  PRT
<213>  Bacillus subtilis

<400>  4

Met Lys Lys Lys Leu Ala Ala Gly Leu Thr Ala Ser Ala Ile Val Gly
1               5                   10                  15

Thr Thr Leu Val Val Thr Pro Ala Glu Ala Ala Thr Ile Lys Val Lys
            20                  25                  30

Ser Gly Asp Ser Leu Trp Lys Leu Ala Gln Thr Tyr Asn Thr Ser Val
        35                  40                  45

Ala Ala Leu Thr Ser Ala Asn His Leu Ser Thr Thr Val Leu Ser Ile
    50                  55                  60

Gly Gln Thr Leu Thr Ile Pro Gly Ser Lys Ser Ser Thr Ser Ser Ser
65                  70                  75                  80

Thr Ser Ser Ser Thr Thr Lys Lys Ser Gly Ser Ser Val Tyr Thr Val
                85                  90                  95

Lys Ser Gly Asp Ser Leu Trp Leu Ile Ala Asn Glu Phe Lys Met Thr
            100                 105                 110

Val Gln Glu Leu Lys Lys Leu Asn Gly Leu Ser Ser Asp Leu Ile Arg
        115                 120                 125

Ala Gly Gln Lys Leu Lys Val Ser Gly Thr Val Ser Ser Ser Ser Ser
    130                 135                 140

Ser Ser Lys Lys Ser Asn Ser Asn Lys Ser Ser Ser Ser Ser Ser Lys
145                 150                 155                 160

Ser Ser Ser Asn Lys Ser Ser Ser Ser Ser Ser Ser Thr Gly Thr Tyr
                165                 170                 175

Lys Val Gln Leu Gly Asp Ser Leu Trp Lys Ile Ala Asn Lys Val Asn
            180                 185                 190

Met Ser Ile Ala Glu Leu Lys Val Leu Asn Asn Leu Lys Ser Asp Thr
        195                 200                 205

Ile Tyr Val Asn Gln Val Leu Lys Thr Lys Ser Ser Gly Ser Asp Thr
    210                 215                 220

Ser Ser Lys Asp Asn Ser Ser Lys Ser Asn Gln Thr Ser Ala Thr Thr
225                 230                 235                 240

Lys Tyr Thr Val Lys Ser Gly Asp Ser Leu Trp Lys Ile Ala Asn Asn
                245                 250                 255

Tyr Asn Leu Thr Val Gln Gln Ile Arg Asn Ile Asn Asn Leu Lys Ser
            260                 265                 270

Asp Val Leu Tyr Val Gly Gln Val Leu Lys Leu Thr Gly Lys Ala Ser
        275                 280                 285

Ser Gly Ser Ser Ser Ser Ser Ser Ser Ser Ser Asn Ala Ser Ser Gly
    290                 295                 300

Thr Thr Thr Thr Tyr Thr Val Lys Ser Gly Asp Ser Leu Trp Val Ile

305                 310                 315                 320

Ala Gln Lys Phe Asn Val Thr Ala Gln Gln Ile Arg Glu Lys Asn Asn
                325                 330                 335

Leu Lys Thr Asp Val Leu Gln Val Gly Gln Lys Leu Val Ile Ser Gly
            340                 345                 350

Lys Ala Ser Ser Ser Ser Ser Ser Gly Ser Ser Asn Thr Thr Ser Ser
        355                 360                 365

Thr Ser Ala Lys Ile Asn Thr Met Ile Ser Ala Ala Lys Ala Gln Leu
    370                 375                 380

Gly Val Pro Tyr Arg Trp Gly Gly Thr Thr Pro Ser Gly Phe Asp Cys
385                 390                 395                 400

Ser Gly Phe Ile Tyr Tyr Val Leu Asn Lys Val Thr Ser Val Ser Arg
                405                 410                 415

Leu Thr Ala Ala Gly Tyr Trp Asn Thr Met Lys Ser Val Ser Gln Pro
            420                 425                 430

Ala Val Gly Asp Phe Val Phe Phe Ser Thr Tyr Lys Ala Gly Pro Ser
        435                 440                 445

His Val Gly Ile Tyr Leu Gly Asn Gly Glu Phe Ile Asn Ala Asn Asp
    450                 455                 460

Ser Gly Val Val Ile Ser Asn Met Asn Asn Ser Tyr Trp Lys Gln Arg
465                 470                 475                 480

Tyr Leu Gly Ala Lys Arg Tyr Phe
                485

<210>  5
<211>  1005
<212>  DNA
<213>  Bacillus subtilis

<220>
<221>  CDS
<222>  (1)..(1005)

<400>  5
atg aaa aag caa atc att aca gct acg aca gca gtt gtt tta gga tcg        48

Met Lys Lys Gln Ile Ile Thr Ala Thr Thr Ala Val Val Leu Gly Ser
1               5                   10                  15

acg tta ttt gca gga gcg gca tct gca caa agc att aag gtg aaa aaa      96
Thr Leu Phe Ala Gly Ala Ala Ser Ala Gln Ser Ile Lys Val Lys Lys
            20                  25                  30

ggc gac acg tta tgg gat ctt tca aga aaa tac gac aca acg atc agt      144
Gly Asp Thr Leu Trp Asp Leu Ser Arg Lys Tyr Asp Thr Thr Ile Ser
        35                  40                  45

aaa att aaa tca gag aac cac ctt cgt tca gac att att tat gtg gga      192
Lys Ile Lys Ser Glu Asn His Leu Arg Ser Asp Ile Ile Tyr Val Gly
    50                  55                  60

caa act tta tcg att aac ggc aaa tct aca agt tca aaa agc agc agt      240
Gln Thr Leu Ser Ile Asn Gly Lys Ser Thr Ser Ser Lys Ser Ser Ser
65                  70                  75                  80

tct tct tcc tct tct tct aca tac aaa gta aag agc ggg gac agc ctt      288
Ser Ser Ser Ser Ser Ser Thr Tyr Lys Val Lys Ser Gly Asp Ser Leu
                85                  90                  95

tgg aaa att tca aaa aaa tac ggc atg aca atc aat gaa ctg aag aag      336
Trp Lys Ile Ser Lys Lys Tyr Gly Met Thr Ile Asn Glu Leu Lys Lys
            100                 105                 110

ctg aat ggc tta aaa tca gat ttg ctt cgt gtt gga caa gtc ctg aaa      384
Leu Asn Gly Leu Lys Ser Asp Leu Leu Arg Val Gly Gln Val Leu Lys
        115                 120                 125

ctg aaa ggt tca act agt tca agc agc tcc agc tca tca aaa gtg tca      432
Leu Lys Gly Ser Thr Ser Ser Ser Ser Ser Ser Ser Ser Lys Val Ser
    130                 135                 140

tcg tct tca act tct act tat aaa gtg aag agc gga gac agc ctt tct      480
Ser Ser Ser Thr Ser Thr Tyr Lys Val Lys Ser Gly Asp Ser Leu Ser
145                 150                 155                 160

aaa att gcg agc aaa tac ggc act acg gtt agc aaa tta aaa agc tta      528
Lys Ile Ala Ser Lys Tyr Gly Thr Thr Val Ser Lys Leu Lys Ser Leu
                165                 170                 175

aac ggc tta aaa tca gat gta atc tat gtc aac caa gta ttg aag gtg      576
Asn Gly Leu Lys Ser Asp Val Ile Tyr Val Asn Gln Val Leu Lys Val
            180                 185                 190

aaa gga aca agc aca agc agc tca aaa cct gct tca tct tca tcg tct      624
Lys Gly Thr Ser Thr Ser Ser Ser Lys Pro Ala Ser Ser Ser Ser Ser
        195                 200                 205

tca agc agc aaa acg tca tct aca tca ctt aat gtg agc aag ctg gtt      672
Ser Ser Ser Lys Thr Ser Ser Thr Ser Leu Asn Val Ser Lys Leu Val
    210                 215                 220

tct gat gca aaa gcg tta gtc gga acg cca tat aaa tgg ggc gga acg      720
Ser Asp Ala Lys Ala Leu Val Gly Thr Pro Tyr Lys Trp Gly Gly Thr
225                 230                 235                 240

aca act tca ggc ttt gac tgc agc gga ttc att tgg tac gta ctg aat 768
Thr Thr Ser Gly Phe Asp Cys Ser Gly Phe Ile Trp Tyr Val Leu Asn
                245                 250                 255

aaa caa aca agt gtg ggc aga aca agc act gca gga tac tgg agt tct 816
Lys Gln Thr Ser Val Gly Arg Thr Ser Thr Ala Gly Tyr Trp Ser Ser
            260                 265                 270

atg aag agc att gcc agc ccg tct gtt ggt gat ttc gtc ttc ttc aca 864
Met Lys Ser Ile Ala Ser Pro Ser Val Gly Asp Phe Val Phe Phe Thr
        275                 280                 285

aca tat aaa tcc ggc cct tct cac atg ggg att tac att gga aac aac 912
Thr Tyr Lys Ser Gly Pro Ser His Met Gly Ile Tyr Ile Gly Asn Asn
    290                 295                 300

agt ttc att cat gca gga tct gac ggc gta caa atc agc agc ctg aac 960
Ser Phe Ile His Ala Gly Ser Asp Gly Val Gln Ile Ser Ser Leu Asn
305                 310                 315                 320

aac agc tac tgg aag cct cgt tac ctc ggt gcg aaa aga ttc taa 1005
Asn Ser Tyr Trp Lys Pro Arg Tyr Leu Gly Ala Lys Arg Phe
                325                 330

<210> 6
<211> 334
<212> PRT
<213> Bacillus subtilis

<400> 6

Met Lys Lys Gln Ile Ile Thr Ala Thr Thr Ala Val Val Leu Gly Ser
1               5                   10                  15

Thr Leu Phe Ala Gly Ala Ala Ser Ala Gln Ser Ile Lys Val Lys Lys
            20                  25                  30

Gly Asp Thr Leu Trp Asp Leu Ser Arg Lys Tyr Asp Thr Thr Ile Ser
        35                  40                  45

Lys Ile Lys Ser Glu Asn His Leu Arg Ser Asp Ile Ile Tyr Val Gly
    50                  55                  60

Gln Thr Leu Ser Ile Asn Gly Lys Ser Thr Ser Ser Lys Ser Ser Ser
65                  70                  75                  80

Ser Ser Ser Ser Ser Ser Thr Tyr Lys Val Lys Ser Gly Asp Ser Leu
                85                  90                  95

Trp Lys Ile Ser Lys Lys Tyr Gly Met Thr Ile Asn Glu Leu Lys Lys
            100                 105                 110

Leu Asn Gly Leu Lys Ser Asp Leu Leu Arg Val Gly Gln Val Leu Lys
        115                 120                 125

Leu Lys Gly Ser Thr Ser Ser Ser Ser Ser Ser Ser Ser Lys Val Ser
    130                 135                 140

Ser Ser Ser Thr Ser Thr Tyr Lys Val Lys Ser Gly Asp Ser Leu Ser
145                 150                 155                 160

Lys Ile Ala Ser Lys Tyr Gly Thr Thr Val Ser Lys Leu Lys Ser Leu
                165                 170                 175

Asn Gly Leu Lys Ser Asp Val Ile Tyr Val Asn Gln Val Leu Lys Val
            180                 185                 190

Lys Gly Thr Ser Thr Ser Ser Ser Lys Pro Ala Ser Ser Ser Ser Ser
        195                 200                 205

Ser Ser Ser Lys Thr Ser Ser Thr Ser Leu Asn Val Ser Lys Leu Val
    210                 215                 220

Ser Asp Ala Lys Ala Leu Val Gly Thr Pro Tyr Lys Trp Gly Gly Thr
225                 230                 235                 240

Thr Thr Ser Gly Phe Asp Cys Ser Gly Phe Ile Trp Tyr Val Leu Asn
                245                 250                 255

Lys Gln Thr Ser Val Gly Arg Thr Ser Thr Ala Gly Tyr Trp Ser Ser
            260                 265                 270

Met Lys Ser Ile Ala Ser Pro Ser Val Gly Asp Phe Val Phe Phe Thr
        275                 280                 285

Thr Tyr Lys Ser Gly Pro Ser His Met Gly Ile Tyr Ile Gly Asn Asn
    290                 295                 300

Ser Phe Ile His Ala Gly Ser Asp Gly Val Gln Ile Ser Ser Leu Asn
305                 310                 315                 320

Asn Ser Tyr Trp Lys Pro Arg Tyr Leu Gly Ala Lys Arg Phe
                325                 330

<210>  7
<211>  18

<212>  DNA
<213>  Artificial

<220>
<221>  source
<223>  /note="Description of artificial sequence: Oligonucleotide as PCR primer
       eprfw1"

<400>  7
ctccggcaga aagggcat                                                    18


<210>  8
<211>  32
<212>  DNA
<213>  Artificial

<220>
<221>  source
<223>  /note="Description of artificial sequence: Oligonucleotide as PCR primer
       eprUPr"

<400>  8
cgttcagccg tacaacaagt ttgcaagaca tg                                    32


<210>  9
<211>  31
<212>  DNA
<213>  Artificial

<220>
<221>  source
<223>  /note="Description of artificial sequence: Oligonucleotide as PCR primer
       eprDNf"

<400>  9
aacttgttgt acggctgaac gccgtcaaac c                                     31


<210>  10
<211>  39
<212>  DNA
<213>  Artificial

<220>
<221>  source
<223>  /note="Description of artificial sequence: Oligonucleotide as PCR primer
       eprrv-repU"

<400>  10
ctgatctcga cttcgttcag acgggtcgta caatggctg                             39


<210>  11
<211>  18
<212>  DNA
<213>  Artificial

<220>
<221>  source
<223>  /note="Description of artificial sequence: Oligonucleotide as PCR primer
      repUfw"

<400>  11
gaacgaagtc gagatcag                                                    18


<210>  12
<211>  21
<212>  DNA
<213>  Artificial

<220>
<221>  source
<223>  /note="Description of artificial sequence: Oligonucleotide as PCR primer
      Cmrv1"

<400>  12
gctgtaatat aaaaaccttc t                                                21


<210>  13
<211>  18
<212>  DNA
<213>  Artificial

<220>
<221>  source
<223>  /note="Description of artificial sequence: Oligonucleotide as PCR primer
      eprfw2"

<400>  13
cgacaccata gctttctg                                                    18


<210>  14
<211>  18
<212>  DNA
<213>  Artificial

<220>
<221>  source
<223>  /note="Description of artificial sequence: Oligonucleotide as PCR primer
      Cmrv2"

<400>  14
aactaacggg gcaggtta                                                    18


<210>  15
<211>  41
<212>  DNA
<213>  Artificial

<220>
<221>  source
<223>  /note="Description of artificial sequence: Oligonucleotide as PCR primer

repUr-CmU"

<400> 15
agaatcaatc ccatggtctc acttttccac tttttgtctt g 41

<210> 16
<211> 41
<212> DNA
<213> Artificial

<220>
<221> source
<223> /note="Description of artificial sequence: Oligonucleotide as PCR primer
CmUf-repU"

<400> 16
gtgagaccat gggattgatt ctaatgaaga aagcagacaa g 41

<210> 17
<211> 20
<212> DNA
<213> Artificial

<220>
<221> source
<223> /note="Description of artificial sequence: Oligonucleotide as PCR primer
wprAfw1"

<400> 17
gggtaattta tctgataggg 20

<210> 18
<211> 33
<212> DNA
<213> Artificial

<220>
<221> source
<223> /note="Description of artificial sequence: Oligonucleotide as PCR primer
wprAUPr"

<400> 18
cttttgcttc ccacaaccga gctgaatttt ctg 33

<210> 19
<211> 34
<212> DNA
<213> Artificial

<220>
<221> source
<223> /note="Description of artificial sequence: Oligonucleotide as PCR primer
wprADNf"

<400> 19

ctcggttgtg ggaagcaaaa gttgttgttg aaaa                                    34


<210>  20
<211>  39
<212>  DNA
<213>  Artificial

<220>
<221>  source
<223>  /note="Description of artificial sequence: Oligonucleotide as PCR primer
       wprArv-repU"

<400>  20
ctgatctcga cttcgttcat cctcattgaa gacggcatc                              39


<210>  21
<211>  20
<212>  DNA
<213>  Artificial

<220>
<221>  source
<223>  /note="Description of artificial sequence: Oligonucleotide as PCR primer
       wprAfw2"

<400>  21
ggaacatata tgacacacct                                                   20


<210>  22
<211>  19
<212>  DNA
<213>  Artificial

<220>
<221>  source
<223>  /note="Description of artificial sequence: Oligonucleotide as PCR primer
       mprfw1"

<400>  22
tgtttggtgt tgagctgtt                                                    19


<210>  23
<211>  34
<212>  DNA
<213>  Artificial

<220>
<221>  source
<223>  /note="Description of artificial sequence: Oligonucleotide as PCR primer
       mprUPr"

<400>  23
ttcgtgtgaa tccattgttt tctgaatctt ggaa                                   34

<210>  24
<211>  33
<212>  DNA
<213>  Artificial

<220>
<221>  source
<223>  /note="Description of artificial sequence: Oligonucleotide as PCR primer
      mprDNf"

<400>  24
gaaaacaatg gattcacacg aacggaggat cgt                                    33


<210>  25
<211>  42
<212>  DNA
<213>  Artificial

<220>
<221>  source
<223>  /note="Description of artificial sequence: Oligonucleotide as PCR primer
      mprrv-repU"

<400>  25
ctgatctcga cttcgttcct cgtaagaaaa aatacctatt tc                          42


<210>  26
<211>  19
<212>  DNA
<213>  Artificial

<220>
<221>  source
<223>  /note="Description of artificial sequence: Oligonucleotide as PCR primer
      mprfw2"

<400>  26
ccttgcgaaa gatagggta                                                    19


<210>  27
<211>  18
<212>  DNA
<213>  Artificial

<220>
<221>  source
<223>  /note="Description of artificial sequence: Oligonucleotide as PCR primer
      nprBfw1"

<400>  27
tttttagcag tggtgctc                                                     18


<210>  28
<211>  32
<212>  DNA

<213>  Artificial

<220>
<221>  source
<223>  /note="Description of artificial sequence: Oligonucleotide as PCR primer
      nprBUPr"

<400>  28
catacgcctt cagtaataga gatgtcttgg tc                                    32


<210>  29
<211>  32
<212>  DNA
<213>  Artificial

<220>
<221>  source
<223>  /note="Description of artificial sequence: Oligonucleotide as PCR primer
      nprBDNf"

<400>  29
ctctattact gaaggcgtat gaggctgtcg gc                                    32


<210>  30
<211>  40
<212>  DNA
<213>  Artificial

<220>
<221>  source
<223>  /note="Description of artificial sequence: Oligonucleotide as PCR primer
      nprBrv-repU"

<400>  30
ctgatctcga cttcgttctt ccaaatgcgc ttcattagga                            40


<210>  31
<211>  18
<212>  DNA
<213>  Artificial

<220>
<221>  source
<223>  /note="Description of artificial sequence: Oligonucleotide as PCR primer
      nprBfw2"

<400>  31
cttttgagcc cgttcctc                                                    18


<210>  32
<211>  19
<212>  DNA
<213>  Artificial

<220>

<221>  source
<223>  /note="Description of artificial sequence: Oligonucleotide as PCR primer
      bprfw1"

<400>  32
tgagattgtg gtgacagtg                                                    19


<210>  33
<211>  33
<212>  DNA
<213>  Artificial

<220>
<221>  source
<223>  /note="Description of artificial sequence: Oligonucleotide as PCR primer
      bprUPr"

<400>  33
ttaagttttc cgctgatgag tctgtttttc gtt                                    33


<210>  34
<211>  35
<212>  DNA
<213>  Artificial

<220>
<221>  source
<223>  /note="Description of artificial sequence: Oligonucleotide as PCR primer
      bprDNf"

<400>  34
gactcatcag cggaaaactt aatatgaaca cagaa                                  35


<210>  35
<211>  41
<212>  DNA
<213>  Artificial

<220>
<221>  source
<223>  /note="Description of artificial sequence: Oligonucleotide as PCR primer
      bprrv-repU"

<400>  35
ctgatctcga cttcgttcgt aatcatgaca ccgttttgaa c                           41


<210>  36
<211>  19
<212>  DNA
<213>  Artificial

<220>
<221>  source
<223>  /note="Description of artificial sequence: Oligonucleotide as PCR primer
      bprfw2"

<400>  36
attgcaaccg gctttatcg                                                    19


<210>  37
<211>  19
<212>  DNA
<213>  Artificial

<220>
<221>  source
<223>  /note="Description of artificial sequence: Oligonucleotide as PCR primer
       nprEfw1"

<400>  37
ttttgaagac gttcggcga                                                    19


<210>  38
<211>  34
<212>  DNA
<213>  Artificial

<220>
<221>  source
<223>  /note="Description of artificial sequence: Oligonucleotide as PCR primer
       nprEUPr"

<400>  38
attgtctgtt gctgaagcag cctggaatgc tgtt                                   34


<210>  39
<211>  34
<212>  DNA
<213>  Artificial

<220>
<221>  source
<223>  /note="Description of artificial sequence: Oligonucleotide as PCR primer
       nprEDNf"

<400>  39
caggctgctt cagcaacaga caatttctta ccta                                   34


<210>  40
<211>  38
<212>  DNA
<213>  Artificial

<220>
<221>  source
<223>  /note="Description of artificial sequence: Oligonucleotide as PCR primer
       nprErv-repU"

<400>  40
ctgatctcga cttcgttccc tctcttaagt aagcgctg                               38

<210> 41
<211> 21
<212> DNA
<213> Artificial

<220>
<221> source
<223> /note="Description of artificial sequence: Oligonucleotide as PCR primer
      nprEfw2"

<400> 41
tctctttgtt gaaaaacgat a                                                21


<210> 42
<211> 19
<212> DNA
<213> Artificial

<220>
<221> source
<223> /note="Description of artificial sequence: Oligonucleotide as PCR primer
      vprfw1"

<400> 42
aaaaacatcc ctccgcttc                                                   19


<210> 43
<211> 32
<212> DNA
<213> Artificial

<220>
<221> source
<223> /note="Description of artificial sequence: Oligonucleotide as PCR primer
      vprUPr"

<400> 43
tcttcggtca atacaagcag aaagcgaatg at                                    32


<210> 44
<211> 32
<212> DNA
<213> Artificial

<220>
<221> source
<223> /note="Description of artificial sequence: Oligonucleotide as PCR primer
      vprDNf"

<400> 44
tctgcttgta ttgaccgaag aacctttcac tg                                    32


<210> 45

<211>  39
<212>  DNA
<213>  Artificial

<220>
<221>  source
<223>  /note="Description of artificial sequence: Oligonucleotide as PCR primer
       vprrv-repU"

<400>  45
ctgatctcga cttcgttctg ctcggctcat ctggagaaa                              39


<210>  46
<211>  18
<212>  DNA
<213>  Artificial

<220>
<221>  source
<223>  /note="Description of artificial sequence: Oligonucleotide as PCR primer
       vprfw2"

<400>  46
tttttggcag gcagcctt                                                     18


<210>  47
<211>  21
<212>  DNA
<213>  Artificial

<220>
<221>  source
<223>  /note="Description of artificial sequence: Oligonucleotide as PCR primer
       aprEfw1"

<400>  47
ctgtttatta tgggccacga a                                                 21


<210>  48
<211>  32
<212>  DNA
<213>  Artificial

<220>
<221>  source
<223>  /note="Description of artificial sequence: Oligonucleotide as PCR primer
       aprEUPr"

<400>  48
gatcagcttg gggttaatca acgtacaagc ag                                     32


<210>  49
<211>  33
<212>  DNA
<213>  Artificial

<220>
<221>  source
<223>  /note="Description of artificial sequence: Oligonucleotide as PCR primer
      aprEDNf"

<400>  49
tgattaaccc caagctgatc cacaattttt tgc                                    33


<210>  50
<211>  39
<212>  DNA
<213>  Artificial

<220>
<221>  source
<223>  /note="Description of artificial sequence: Oligonucleotide as PCR primer
      aprErv-repu"

<400>  50
ctgatctcga cttcgttctg attttccaaa cgagctttc                              39


<210>  51
<211>  21
<212>  DNA
<213>  Artificial

<220>
<221>  source
<223>  /note="Description of artificial sequence: Oligonucleotide as PCR primer
      aprEfw2"

<400>  51
atgggccatt atgtcatgaa g                                                 21


<210>  52
<211>  38
<212>  DNA
<213>  Artificial

<220>
<221>  source
<223>  /note="Description of artificial sequence: Oligonucleotide as PCR primer
      yoeBSal-F-2"

<400>  52
gcgcagatct gtcgactaag agaaagagat tttgaagg                               38


<210>  53
<211>  31
<212>  DNA
<213>  Artificial

<220>
<221>  source

<223>  /note="Description of artificial sequence: Oligonucleotide as PCR primer
      yoeBSph-R"

<400>  53
gcgcgcatgc ttatataact gcgtcaaatt g                                    31


<210>  54
<211>  1245
<212>  DNA
<213>  Bacillus subtilis


<220>
<221>  CDS
<222>  (1)..(1245)

<400>  54
atg aaa aag aag att gta gcc ggc ttg gct gtt tct gca gtt gtt ggg       48
tcg tcg atg gcc gca gca ccc gcg gaa gca aaa acg ata aaa gta aaa       96
agc ggt gac tct ctt tgg aaa ctt tcc cgc cag tat gat acg aca ata      144
tca gct ctg aaa tcc gaa aac aaa ttg aaa tca acg gtg ctt tat gtc      192
gga caa agt ctt aag gtt cct gaa agc agc aaa aaa agc acg aca tcc      240
cca tct aat tca agc aaa aca tca aca tac aca gtt gca tat ggt gat      288
tcc cta tgg atg att gct aaa aat cat aaa atg agt gtt tct gag ctt      336
aaa agc tta aac agc ctg agc agt gac ctg att cgt ccg ggg caa aag      384
ctt aag atc aaa ggg aca agc agt tca agc gga tca aat gga tca aag      432
aaa aac agc ggt tcc aat tct tca ggt tct tca aaa agt aca tat acg      480
gtc aag ctt ggt gac tca ctg tgg aaa atc gca aac agc tta aat atg      528
act gta gcc gaa tta aaa acc tta aat ggc tta aca tca gat aca ctt      576
tat cct aaa cag gtg ctc aaa ata aaa gga agc agt tca cct aag aac      624
gga aac agc ggt tcc aaa aag ccg tca aac tcc aat ccc tca aaa aca      672
acg acg tac aag gta aaa gca ggc gat tcg ctg tgg aaa att gca aat      720
cgg ctc gga gtc act gtc caa agc atc cgt gat aaa aac aat tta tca      768
tcg gat gtg ttg caa atc ggg cag gtt ctg act att tcg gga gca tca      816
aaa tct aat cct tca aat ccg aca aag cca aca aag cca aaa gat aac      864
tca ggg tca aac att caa ata ggt tcg aag att gac aga atg ata acg      912
gaa gcg aaa aaa tat gtc gga gtg cct tac cga tgg ggc gga aac acg      960
cct gcc ggt ttt gat tgc agc ggc ttc att tat tac ctc att aat aat     1008
gta tca tct ata tca aga ttg agt acg gca ggg tat tgg aat gtc atg     1056
cag aaa gtc agc cag ccg tca gta gga gac ttt gtc ttc ttt acc aca     1104
tat aaa tcc ggc ccg tct cac atg ggg att tat cta ggc ggc ggt gat     1152
ttt att cat gca agc tca tcg ggc gtc gac att tcc aat ctc agc aat     1200
tcg tat tgg aaa cag cgg tac ttg ggc gca aga agt tat ttt taa          1245

<210>  55
<211>  414
<212>  PRT
<213>  Bacillus subtilis

<400>  55

<210> 56
<211> 1422

<212>  DNA
<213>  Bacillus subtilis

<220>
<221>  CDS
<222>  (1)..(1422)

<400>  56
gtg aga aag agt tta att aca ctt ggt ttg gct tcc gtc atc ggg aca      48
Val Arg Lys Ser Leu Ile Thr Leu Gly Leu Ala Ser Val Ile Gly Thr
1               5                   10                  15

agc agt ttt ttg atc cca ttt aca agt aaa act gca tcg gcg gaa aca      96
Ser Ser Phe Leu Ile Pro Phe Thr Ser Lys Thr Ala Ser Ala Glu Thr
            20                  25                  30

tta gat gaa aag aaa caa aaa atc gaa agc aag caa tct gag gtt gct      144
Leu Asp Glu Lys Lys Gln Lys Ile Glu Ser Lys Gln Ser Glu Val Ala
        35                  40                  45

tcc agc att gaa gcg aag gaa aaa gaa tta acc gag ctt cag gaa aat      192
Ser Ser Ile Glu Ala Lys Glu Lys Glu Leu Thr Glu Leu Gln Glu Asn
    50                  55                  60

caa tca aag att gaa aaa gaa ctg aaa gac att aac gat aag gcg ctt      240
Gln Ser Lys Ile Glu Lys Glu Leu Lys Asp Ile Asn Asp Lys Ala Leu
65                  70                  75                  80

gat aca agc aac aag att gaa gat aaa aaa gaa gaa aac gat aaa aca      288
Asp Thr Ser Asn Lys Ile Glu Asp Lys Lys Glu Glu Asn Asp Lys Thr
                85                  90                  95

aaa gaa gaa atc aaa aaa ctg aaa aaa gag att aaa gaa act gaa gca      336
Lys Glu Glu Ile Lys Lys Leu Lys Lys Glu Ile Lys Glu Thr Glu Ala
            100                 105                 110

cgc atc gaa aaa cgc aat gaa atc ctg aaa aaa cgc gtt cgt tct tta      384
Arg Ile Glu Lys Arg Asn Glu Ile Leu Lys Lys Arg Val Arg Ser Leu
        115                 120                 125

cag gaa agc ggc gga tct caa gga tac ata gat gtt ctt tta gga tca      432
Gln Glu Ser Gly Gly Ser Gln Gly Tyr Ile Asp Val Leu Leu Gly Ser
    130                 135                 140

aca agc ttt ggt gac ttt atc tct cgt gcg act gcg gtt tca tct att      480
Thr Ser Phe Gly Asp Phe Ile Ser Arg Ala Thr Ala Val Ser Ser Ile
145                 150                 155                 160

gtg gac gca gac aaa gat tta atc aaa cag caa gag cag gat aaa gcg      528
Val Asp Ala Asp Lys Asp Leu Ile Lys Gln Gln Glu Gln Asp Lys Ala
                165                 170                 175

aag ctc gaa gat tct gaa gcg gat ttg aat gac aag ctg aaa gaa gtt      576
Lys Leu Glu Asp Ser Glu Ala Asp Leu Asn Asp Lys Leu Lys Glu Val
            180                 185                 190

caa gct gcc ttg gct aaa tta gaa aca atg caa aaa gac ctt gat aaa      624
Gln Ala Ala Leu Ala Lys Leu Glu Thr Met Gln Lys Asp Leu Asp Lys

        195                 200                 205

cag ctt aat gaa aaa gac aag ctt ttc gac gaa gca aaa gca agc caa 672
Gln Leu Asn Glu Lys Asp Lys Leu Phe Asp Glu Ala Lys Ala Ser Gln
    210                 215                 220

aag aaa acg gct aaa gcg att tct gaa tta aaa tca gag gcg tct gaa 720
Lys Lys Thr Ala Lys Ala Ile Ser Glu Leu Lys Ser Glu Ala Ser Glu
225                 230                 235                 240

ctt gca aat caa aaa gca aat act gaa gct gaa caa gca cgc atc aaa 768
Leu Ala Asn Gln Lys Ala Asn Thr Glu Ala Glu Gln Ala Arg Ile Lys
                245                 250                 255

aaa gaa caa gaa gca gcg gct gct ttg atc aaa aag cag gaa gaa gca 816
Lys Glu Gln Glu Ala Ala Ala Ala Leu Ile Lys Lys Gln Glu Glu Ala
            260                 265                 270

caa aaa gca tct gat gaa aca caa aca gat gac agt caa acg gcg aca 864
Gln Lys Ala Ser Asp Glu Thr Gln Thr Asp Asp Ser Gln Thr Ala Thr
        275                 280                 285

act gaa tca tca aaa gca agc tca tct gat gat tct tca gac aat tct 912
Thr Glu Ser Ser Lys Ala Ser Ser Ser Asp Asp Ser Ser Asp Asn Ser
    290                 295                 300

tca gac aat tct tct aac ggt tca tca aac agt tcg tca aac ggc tca 960
Ser Asp Asn Ser Ser Asn Gly Ser Ser Asn Ser Ser Ser Asn Gly Ser
305                 310                 315                 320

tct tct aag aag agc agc ggc tca aac agc aat tca ggc ggc act gtt 1008
Ser Ser Lys Lys Ser Ser Gly Ser Asn Ser Asn Ser Gly Gly Thr Val
                325                 330                 335

atc agc aac tct ggc gga att gaa ggc gcg atc agc gtt ggt tca agc 1056
Ile Ser Asn Ser Gly Gly Ile Glu Gly Ala Ile Ser Val Gly Ser Ser
            340                 345                 350

att gta gga caa tct ccg tac aag ttt ggc ggc gga cgc act cag tct 1104
Ile Val Gly Gln Ser Pro Tyr Lys Phe Gly Gly Gly Arg Thr Gln Ser
        355                 360                 365

gat atc aac aac cgt att ttt gac tgc tca tca ttc gta cgc tgg gca 1152
Asp Ile Asn Asn Arg Ile Phe Asp Cys Ser Ser Phe Val Arg Trp Ala
    370                 375                 380

tac gct tct gca ggt gtt aac ctt gga cct gta ggc gga aca aca act 1200
Tyr Ala Ser Ala Gly Val Asn Leu Gly Pro Val Gly Gly Thr Thr Thr
385                 390                 395                 400

gat acg tta gtt ggc aga gga caa gct gtc agc gcg tct gaa atg aaa 1248
Asp Thr Leu Val Gly Arg Gly Gln Ala Val Ser Ala Ser Glu Met Lys
                405                 410                 415

cgc gga gac ctt gtg ttc ttt gac act tac aaa aca aat gga cac gta 1296
Arg Gly Asp Leu Val Phe Phe Asp Thr Tyr Lys Thr Asn Gly His Val
            420                 425                 430

gga atc tac tta gga aac ggt act ttc cta aac gac aat aca tct cat 1344

Gly Ile Tyr Leu Gly Asn Gly Thr Phe Leu Asn Asp Asn Thr Ser His
        435                 440                 445

ggc gta tct gtt gac tct atg agc aat cct tac tgg aaa gca gca ttt      1392
Gly Val Ser Val Asp Ser Met Ser Asn Pro Tyr Trp Lys Ala Ala Phe
    450                 455                 460

aaa ggt gtt gta aga cgt gtt gtt caa taa                              1422
Lys Gly Val Val Arg Arg Val Val Gln
465                 470

<210>  57
<211>  473
<212>  PRT
<213>  Bacillus subtilis

<400>  57

Val Arg Lys Ser Leu Ile Thr Leu Gly Leu Ala Ser Val Ile Gly Thr
1               5                   10                  15

Ser Ser Phe Leu Ile Pro Phe Thr Ser Lys Thr Ala Ser Ala Glu Thr
            20                  25                  30

Leu Asp Glu Lys Lys Gln Lys Ile Glu Ser Lys Gln Ser Glu Val Ala
        35                  40                  45

Ser Ser Ile Glu Ala Lys Glu Lys Glu Leu Thr Glu Leu Gln Glu Asn
    50                  55                  60

Gln Ser Lys Ile Glu Lys Glu Leu Lys Asp Ile Asn Asp Lys Ala Leu
65                  70                  75                  80

Asp Thr Ser Asn Lys Ile Glu Asp Lys Lys Glu Glu Asn Asp Lys Thr
                85                  90                  95

Lys Glu Glu Ile Lys Lys Leu Lys Lys Glu Ile Lys Glu Thr Glu Ala
            100                 105                 110

Arg Ile Glu Lys Arg Asn Glu Ile Leu Lys Lys Arg Val Arg Ser Leu
        115                 120                 125

Gln Glu Ser Gly Gly Ser Gln Gly Tyr Ile Asp Val Leu Leu Gly Ser
    130                 135                 140

Thr Ser Phe Gly Asp Phe Ile Ser Arg Ala Thr Ala Val Ser Ser Ile
145                 150                 155                 160

Val Asp Ala Asp Lys Asp Leu Ile Lys Gln Gln Glu Gln Asp Lys Ala
                165                 170                 175

Lys Leu Glu Asp Ser Glu Ala Asp Leu Asn Asp Lys Leu Lys Glu Val
            180                 185                 190

Gln Ala Ala Leu Ala Lys Leu Glu Thr Met Gln Lys Asp Leu Asp Lys
        195                 200                 205

Gln Leu Asn Glu Lys Asp Lys Leu Phe Asp Glu Ala Lys Ala Ser Gln
    210                 215                 220

Lys Lys Thr Ala Lys Ala Ile Ser Glu Leu Lys Ser Glu Ala Ser Glu
225                 230                 235                 240

Leu Ala Asn Gln Lys Ala Asn Thr Glu Ala Glu Gln Ala Arg Ile Lys
                245                 250                 255

Lys Glu Gln Glu Ala Ala Ala Ala Leu Ile Lys Lys Gln Glu Glu Ala
            260                 265                 270

Gln Lys Ala Ser Asp Glu Thr Gln Thr Asp Asp Ser Gln Thr Ala Thr
        275                 280                 285

Thr Glu Ser Ser Lys Ala Ser Ser Ser Asp Asp Ser Ser Asp Asn Ser
    290                 295                 300

Ser Asp Asn Ser Ser Asn Gly Ser Ser Asn Ser Ser Ser Asn Gly Ser
305                 310                 315                 320

Ser Ser Lys Lys Ser Ser Gly Ser Asn Ser Asn Ser Gly Gly Thr Val
                325                 330                 335

Ile Ser Asn Ser Gly Gly Ile Glu Gly Ala Ile Ser Val Gly Ser Ser
            340                 345                 350

Ile Val Gly Gln Ser Pro Tyr Lys Phe Gly Gly Gly Arg Thr Gln Ser
        355                 360                 365

Asp Ile Asn Asn Arg Ile Phe Asp Cys Ser Ser Phe Val Arg Trp Ala
    370                 375                 380

Tyr Ala Ser Ala Gly Val Asn Leu Gly Pro Val Gly Gly Thr Thr Thr
385                 390                 395                 400

Asp Thr Leu Val Gly Arg Gly Gln Ala Val Ser Ala Ser Glu Met Lys
                405                 410                 415

Arg Gly Asp Leu Val Phe Phe Asp Thr Tyr Lys Thr Asn Gly His Val
            420                 425                 430

Gly Ile Tyr Leu Gly Asn Gly Thr Phe Leu Asn Asp Asn Thr Ser His
        435                 440                 445

Gly Val Ser Val Asp Ser Met Ser Asn Pro Tyr Trp Lys Ala Ala Phe
    450                 455                 460

Lys Gly Val Val Arg Arg Val Val Gln
465                 470

<210>  58
<211>  990
<212>  DNA
<213>  Bacillus subtilis

<220>
<221>  CDS
<222>  (1)..(990)

<400>  58
atg ata agt aaa aag gta gtt ctt cct ttg gta ttc tcg gct cct ttt       48
Met Ile Ser Lys Lys Val Val Leu Pro Leu Val Phe Ser Ala Pro Phe
1               5                   10                  15

atc ttc ttt ttt gtt tta tgt atc gta gtg gtt atg acg att tca aga       96
Ile Phe Phe Phe Val Leu Cys Ile Val Val Val Met Thr Ile Ser Arg
            20                  25                  30

gaa aat caa gtc ggg gac gat ttt ata ggc ggc ggg gat ggc gaa tat      144
Glu Asn Gln Val Gly Asp Asp Phe Ile Gly Gly Gly Asp Gly Glu Tyr
        35                  40                  45

gaa aca gtt gga att gcg ccg gaa gtg gaa cga ttt aga gca gta ttt      192
Glu Thr Val Gly Ile Ala Pro Glu Val Glu Arg Phe Arg Ala Val Phe
    50                  55                  60

gag aaa tat gcc cgt caa gaa ggc gtt ttt gac caa gtg aat att att      240
Glu Lys Tyr Ala Arg Gln Glu Gly Val Phe Asp Gln Val Asn Ile Ile
65                  70                  75                  80

atg gcg ctc acc atg caa gaa agt ggt ggg cgt tct tta gat atc atg      288
Met Ala Leu Thr Met Gln Glu Ser Gly Gly Arg Ser Leu Asp Ile Met
                85                  90                  95

caa agt tcc gaa agt atc ggc tta cct ccc aac tcc ata act gac cca      336
Gln Ser Ser Glu Ser Ile Gly Leu Pro Pro Asn Ser Ile Thr Asp Pro
            100                 105                 110

gaa cgt tca att gaa gtc ggc att aaa cat ttt aag aag gtt ttt aaa 384
Glu Arg Ser Ile Glu Val Gly Ile Lys His Phe Lys Lys Val Phe Lys
        115                 120                 125

cag gct ggc ggt gat gtt cgt tta acc tta cag gca tac aac ttt ggt 432
Gln Ala Gly Gly Asp Val Arg Leu Thr Leu Gln Ala Tyr Asn Phe Gly
    130                 135                 140

tct gga ttt ata gac tat gtc aaa aag aat ggc ggg aaa tac aca aag 480
Ser Gly Phe Ile Asp Tyr Val Lys Lys Asn Gly Gly Lys Tyr Thr Lys
145                 150                 155                 160

aaa tta gca ctt gat ttc agc cga ttg caa gct ttt aaa atg ggc tgg 528
Lys Leu Ala Leu Asp Phe Ser Arg Leu Gln Ala Phe Lys Met Gly Trp
                165                 170                 175

aaa agc tac ggt gac cca agt tat gtt gac cat gtc atg agg tat gtg 576
Lys Ser Tyr Gly Asp Pro Ser Tyr Val Asp His Val Met Arg Tyr Val
            180                 185                 190

aaa ggc tct gat aaa aat gtg aag cct gtg aaa ggc tct atg gat ttc 624
Lys Gly Ser Asp Lys Asn Val Lys Pro Val Lys Gly Ser Met Asp Phe
        195                 200                 205

tat gaa acg gtc atg aaa gaa gct ttg aaa tat gaa ggg caa ccg tat 672
Tyr Glu Thr Val Met Lys Glu Ala Leu Lys Tyr Glu Gly Gln Pro Tyr
    210                 215                 220

gcc tgg gga ggt tca aac ccg gaa act gga ttt gat tgt tct ggc ctt 720
Ala Trp Gly Gly Ser Asn Pro Glu Thr Gly Phe Asp Cys Ser Gly Leu
225                 230                 235                 240

gtt cag tgg tct ttt gcg aaa gct gga atc act ctt cct aga aca gca 768
Val Gln Trp Ser Phe Ala Lys Ala Gly Ile Thr Leu Pro Arg Thr Ala
                245                 250                 255

caa gaa caa cat ggg gca acc aag aaa ata agt gaa aaa gaa gcg act 816
Gln Glu Gln His Gly Ala Thr Lys Lys Ile Ser Glu Lys Glu Ala Thr
            260                 265                 270

gcg ggt gac tta gtg ttc ttt ggc ggc aca tac gaa gga aaa gcc att 864
Ala Gly Asp Leu Val Phe Phe Gly Gly Thr Tyr Glu Gly Lys Ala Ile
        275                 280                 285

act cac gtc ggc atc tat gta ggc aat ggc aga atg ttt aat tca aat 912
Thr His Val Gly Ile Tyr Val Gly Asn Gly Arg Met Phe Asn Ser Asn
    290                 295                 300

gac agt gga atc caa tat tct gat ctg aaa tca ggt tac tgg aga gat 960
Asp Ser Gly Ile Gln Tyr Ser Asp Leu Lys Ser Gly Tyr Trp Arg Asp
305                 310                 315                 320

cac ctt gta tct ttt gga cgc att aaa taa 990
His Leu Val Ser Phe Gly Arg Ile Lys
                325

<210> 59

<211>  329
<212>  PRT
<213>  Bacillus subtilis

<400>  59

Met Ile Ser Lys Lys Val Val Leu Pro Leu Val Phe Ser Ala Pro Phe
1               5                   10                  15

Ile Phe Phe Phe Val Leu Cys Ile Val Val Val Met Thr Ile Ser Arg
            20                  25                  30

Glu Asn Gln Val Gly Asp Asp Phe Ile Gly Gly Gly Asp Gly Glu Tyr
        35                  40                  45

Glu Thr Val Gly Ile Ala Pro Glu Val Glu Arg Phe Arg Ala Val Phe
    50                  55                  60

Glu Lys Tyr Ala Arg Gln Glu Gly Val Phe Asp Gln Val Asn Ile Ile
65                  70                  75                  80

Met Ala Leu Thr Met Gln Glu Ser Gly Gly Arg Ser Leu Asp Ile Met
                85                  90                  95

Gln Ser Ser Glu Ser Ile Gly Leu Pro Pro Asn Ser Ile Thr Asp Pro
            100                 105                 110

Glu Arg Ser Ile Glu Val Gly Ile Lys His Phe Lys Lys Val Phe Lys
        115                 120                 125

Gln Ala Gly Gly Asp Val Arg Leu Thr Leu Gln Ala Tyr Asn Phe Gly
    130                 135                 140

Ser Gly Phe Ile Asp Tyr Val Lys Lys Asn Gly Gly Lys Tyr Thr Lys
145                 150                 155                 160

Lys Leu Ala Leu Asp Phe Ser Arg Leu Gln Ala Phe Lys Met Gly Trp
                165                 170                 175

Lys Ser Tyr Gly Asp Pro Ser Tyr Val Asp His Val Met Arg Tyr Val
            180                 185                 190

Lys Gly Ser Asp Lys Asn Val Lys Pro Val Lys Gly Ser Met Asp Phe
        195                 200                 205

Tyr Glu Thr Val Met Lys Glu Ala Leu Lys Tyr Glu Gly Gln Pro Tyr

210                 215                 220

Ala Trp Gly Gly Ser Asn Pro Glu Thr Gly Phe Asp Cys Ser Gly Leu
225                 230                 235                 240

Val Gln Trp Ser Phe Ala Lys Ala Gly Ile Thr Leu Pro Arg Thr Ala
                245                 250                 255

Gln Glu Gln His Gly Ala Thr Lys Lys Ile Ser Glu Lys Glu Ala Thr
            260                 265                 270

Ala Gly Asp Leu Val Phe Phe Gly Gly Thr Tyr Glu Gly Lys Ala Ile
        275                 280                 285

Thr His Val Gly Ile Tyr Val Gly Asn Gly Arg Met Phe Asn Ser Asn
    290                 295                 300

Asp Ser Gly Ile Gln Tyr Ser Asp Leu Lys Ser Gly Tyr Trp Arg Asp
305                 310                 315                 320

His Leu Val Ser Phe Gly Arg Ile Lys
                325

<210>  60
<211>  31
<212>  DNA
<213>  Artificial

<220>
<221>  source
<223>  /note="Description of artificial sequence: Oligonucleotide as PCR primer
       BF-YOJL"

<400>  60
gcgcggatcc tcaaacattc aaataggttc g                                    31

<210>  61
<211>  29
<212>  DNA
<213>  Artificial

<220>
<221>  source
<223>  /note="Description of artificial sequence: Oligonucleotide as PCR primer
       KR-YOJL"

<400>  61
gcgcggtacc ggtcaatcat tgtctggta                                       29

<210>  62
<211>  28
<212>  DNA
<213>  Artificial

<220>
<221>  source
<223>  /note="Description of artificial sequence: Oligonucleotide as PCR primer
       BF-YVCE"

<400>  62
gcgcggatcc gaaggcgcga tcagcgtt                                          28


<210>  63
<211>  31
<212>  DNA
<213>  Artificial

<220>
<221>  source
<223>  /note="Description of artificial sequence: Oligonucleotide as PCR primer
       KR-YVCE"

<400>  63
gcgcggtacc gctcaacacg attaaatgta t                                      31


<210>  64
<211>  31
<212>  DNA
<213>  Artificial

<220>
<221>  source
<223>  /note="Description of artificial sequence: Oligonucleotide as PCR primer
       BF-YDDH"

<400>  64
gcgcggatcc gatttctatg aaacggtcat g                                      31


<210>  65
<211>  31
<212>  DNA
<213>  Artificial

<220>
<221>  source
<223>  /note="Description of artificial sequence: Oligonucleotide as PCR primer
       KR-YDDH"

<400>  65
gcgcggtacc cactcctagt tatttaatgc g                                      31


<210>  66
<211>  1242
<212>  DNA

<213>  Bacillus subtilis

<220>
<221>  CDS
<222>  (1)..(1242)

<400>  66
gtg aac aca ctg gca aac tgg aag aag ttt ttg ctt gtg gcg gtt atc      48
Val Asn Thr Leu Ala Asn Trp Lys Lys Phe Leu Leu Val Ala Val Ile
1               5                   10                  15

att tgt ttt ttg gtt cca att atg aca aaa gcg gag att gcg gaa gct      96
Ile Cys Phe Leu Val Pro Ile Met Thr Lys Ala Glu Ile Ala Glu Ala
            20                  25                  30

gat aca tca tca gaa ttg att gtc agc gaa gca aaa aac ctg ctt gga      144
Asp Thr Ser Ser Glu Leu Ile Val Ser Glu Ala Lys Asn Leu Leu Gly
        35                  40                  45

tat cag tat aaa tat ggc ggg gaa acg ccg aaa gag ggt ttc gat cca      192
Tyr Gln Tyr Lys Tyr Gly Gly Glu Thr Pro Lys Glu Gly Phe Asp Pro
    50                  55                  60

tca gga ttg ata caa tat gtg ttc agt aag gct gat att cat ctg ccg      240
Ser Gly Leu Ile Gln Tyr Val Phe Ser Lys Ala Asp Ile His Leu Pro
65                  70                  75                  80

aga tct gta aac gac cag tat aaa atc gga aca gct gta aaa ccg gaa      288
Arg Ser Val Asn Asp Gln Tyr Lys Ile Gly Thr Ala Val Lys Pro Glu
                85                  90                  95

aac ctg aag ccg ggt gat att ttg ttt ttc aag aaa gag gga agc acc      336
Asn Leu Lys Pro Gly Asp Ile Leu Phe Phe Lys Lys Glu Gly Ser Thr
            100                 105                 110

ggc act gtt ccg aca cat gac gcc ctt tat atc gga gac ggc caa atg      384
Gly Thr Val Pro Thr His Asp Ala Leu Tyr Ile Gly Asp Gly Gln Met
        115                 120                 125

gtt cac agt aca cag tca aaa ggg gtt atc atc acc aat tac aaa aaa      432
Val His Ser Thr Gln Ser Lys Gly Val Ile Ile Thr Asn Tyr Lys Lys
    130                 135                 140

agc agc tat tgg agc gga act tat atc ggg gcg aga cga atc gct gcc      480
Ser Ser Tyr Trp Ser Gly Thr Tyr Ile Gly Ala Arg Arg Ile Ala Ala
145                 150                 155                 160

gat ccg gca acg gct gat gtt cct gtc gtt cag gag gcc gaa aaa tat      528
Asp Pro Ala Thr Ala Asp Val Pro Val Val Gln Glu Ala Glu Lys Tyr
                165                 170                 175

atc ggt gtc cca tat gtg ttt ggc gga agc acg ccg tca gag ggc ttt      576
Ile Gly Val Pro Tyr Val Phe Gly Gly Ser Thr Pro Ser Glu Gly Phe
            180                 185                 190

gat tgc tcg ggg ctt gtg caa tat gtg ttt caa cag gca ctc ggc att      624
Asp Cys Ser Gly Leu Val Gln Tyr Val Phe Gln Gln Ala Leu Gly Ile
        195                 200                 205

tat cta ccg cga tca gcc gaa cag cag tgg gca gtg ggc gag aag gta     672
Tyr Leu Pro Arg Ser Ala Glu Gln Gln Trp Ala Val Gly Glu Lys Val
    210                 215                 220

gcc cct cag aac ata aag cct ggt gat gtc gtc tat ttc agc aat acg     720
Ala Pro Gln Asn Ile Lys Pro Gly Asp Val Val Tyr Phe Ser Asn Thr
225                 230                 235                 240

tat aaa acg gga att tca cat gca ggc att tat gcg ggc gca ggc agg     768
Tyr Lys Thr Gly Ile Ser His Ala Gly Ile Tyr Ala Gly Ala Gly Arg
                245                 250                 255

ttc att cag gca agc cgg tca gaa aaa gta acc att tcc tat ttg tca     816
Phe Ile Gln Ala Ser Arg Ser Glu Lys Val Thr Ile Ser Tyr Leu Ser
            260                 265                 270

gag gat tac tgg aaa tcg aag atg acg ggt att cgc cga ttt gac aac     864
Glu Asp Tyr Trp Lys Ser Lys Met Thr Gly Ile Arg Arg Phe Asp Asn
        275                 280                 285

ctg aca atc ccg aaa gaa aat ccg att gtt tcc gaa gcg acg ctt tat     912
Leu Thr Ile Pro Lys Glu Asn Pro Ile Val Ser Glu Ala Thr Leu Tyr
    290                 295                 300

gtc gga gaa gtg cct tac aaa cag ggc gga gta aca cct gag acg gga     960
Val Gly Glu Val Pro Tyr Lys Gln Gly Gly Val Thr Pro Glu Thr Gly
305                 310                 315                 320

ttt gat aca gct gga ttt gtc caa tat gta tac cag aaa gca gcc ggt     1008
Phe Asp Thr Ala Gly Phe Val Gln Tyr Val Tyr Gln Lys Ala Ala Gly
                325                 330                 335

att tcc ctg cct cga tac gca aca agc cag tac aat gcc gga act aag     1056
Ile Ser Leu Pro Arg Tyr Ala Thr Ser Gln Tyr Asn Ala Gly Thr Lys
            340                 345                 350

att gag aag gcg gac ctg aag ccg gga gac att gtg ttc ttt caa tca     1104
Ile Glu Lys Ala Asp Leu Lys Pro Gly Asp Ile Val Phe Phe Gln Ser
        355                 360                 365

aca agc tta aat ccc tcc atc tat atc gga aac gga caa gtt gtt cat     1152
Thr Ser Leu Asn Pro Ser Ile Tyr Ile Gly Asn Gly Gln Val Val His
    370                 375                 380

gtc aca tta tca aac ggc gtg acc att acc aat atg aac acg agc aca     1200
Val Thr Leu Ser Asn Gly Val Thr Ile Thr Asn Met Asn Thr Ser Thr
385                 390                 395                 400

tat tgg aag gat aaa tac gca gga agt ata cgg gtg caa taa             1242
Tyr Trp Lys Asp Lys Tyr Ala Gly Ser Ile Arg Val Gln
                405                 410

<210>  67
<211>  413
<212>  PRT
<213>  Bacillus subtilis

<400> 67

Val Asn Thr Leu Ala Asn Trp Lys Lys Phe Leu Leu Val Ala Val Ile
1               5                   10                  15

Ile Cys Phe Leu Val Pro Ile Met Thr Lys Ala Glu Ile Ala Glu Ala
            20                  25                  30

Asp Thr Ser Ser Glu Leu Ile Val Ser Glu Ala Lys Asn Leu Leu Gly
        35                  40                  45

Tyr Gln Tyr Lys Tyr Gly Gly Glu Thr Pro Lys Glu Gly Phe Asp Pro
    50                  55                  60

Ser Gly Leu Ile Gln Tyr Val Phe Ser Lys Ala Asp Ile His Leu Pro
65                  70                  75                  80

Arg Ser Val Asn Asp Gln Tyr Lys Ile Gly Thr Ala Val Lys Pro Glu
                85                  90                  95

Asn Leu Lys Pro Gly Asp Ile Leu Phe Phe Lys Lys Glu Gly Ser Thr
            100                 105                 110

Gly Thr Val Pro Thr His Asp Ala Leu Tyr Ile Gly Asp Gly Gln Met
        115                 120                 125

Val His Ser Thr Gln Ser Lys Gly Val Ile Ile Thr Asn Tyr Lys Lys
    130                 135                 140

Ser Ser Tyr Trp Ser Gly Thr Tyr Ile Gly Ala Arg Arg Ile Ala Ala
145                 150                 155                 160

Asp Pro Ala Thr Ala Asp Val Pro Val Val Gln Glu Ala Glu Lys Tyr
                165                 170                 175

Ile Gly Val Pro Tyr Val Phe Gly Gly Ser Thr Pro Ser Glu Gly Phe
            180                 185                 190

Asp Cys Ser Gly Leu Val Gln Tyr Val Phe Gln Gln Ala Leu Gly Ile
        195                 200                 205

Tyr Leu Pro Arg Ser Ala Glu Gln Gln Trp Ala Val Gly Glu Lys Val
    210                 215                 220

Ala Pro Gln Asn Ile Lys Pro Gly Asp Val Val Tyr Phe Ser Asn Thr

225                 230                 235                 240

Tyr Lys Thr Gly Ile Ser His Ala Gly Ile Tyr Ala Gly Ala Gly Arg
                245                 250                 255

Phe Ile Gln Ala Ser Arg Ser Glu Lys Val Thr Ile Ser Tyr Leu Ser
            260                 265                 270

Glu Asp Tyr Trp Lys Ser Lys Met Thr Gly Ile Arg Arg Phe Asp Asn
        275                 280                 285

Leu Thr Ile Pro Lys Glu Asn Pro Ile Val Ser Glu Ala Thr Leu Tyr
    290                 295                 300

Val Gly Glu Val Pro Tyr Lys Gln Gly Gly Val Thr Pro Glu Thr Gly
305                 310                 315                 320

Phe Asp Thr Ala Gly Phe Val Gln Tyr Val Tyr Gln Lys Ala Ala Gly
                325                 330                 335

Ile Ser Leu Pro Arg Tyr Ala Thr Ser Gln Tyr Asn Ala Gly Thr Lys
            340                 345                 350

Ile Glu Lys Ala Asp Leu Lys Pro Gly Asp Ile Val Phe Phe Gln Ser
        355                 360                 365

Thr Ser Leu Asn Pro Ser Ile Tyr Ile Gly Asn Gly Gln Val Val His
    370                 375                 380

Val Thr Leu Ser Asn Gly Val Thr Ile Thr Asn Met Asn Thr Ser Thr
385                 390                 395                 400

Tyr Trp Lys Asp Lys Tyr Ala Gly Ser Ile Arg Val Gln
                405                 410

<210>  68
<211>  31
<212>  DNA
<213>  Artificial

<220>
<221>  source
<223>  /note="Description of artificial sequence: Oligonucleotide as PCR primer
       YwtD-FL-Fw"

<400>  68
gcgcggtacc gatacatcat cagaattgat t                                    31

<210>  69
<211>  30
<212>  DNA
<213>  Artificial

<220>
<221>  source
<223>  /note="Description of artificial sequence: Oligonucleotide as PCR primer
       YwtD-FL-Rv"

<400>  69
gcgcctgcag ttattgcacc cgtatacttc                                        30

## Description

### Technical Field

The present invention relates to a lytic enzyme inhibitor capable of inhibiting lytic activity of a given Bacillus subtilis-derived lytic enzyme, a lysis inhibitor capable of inhibiting microbial lysis, a microorganism whose lysis is inhibited, an inhibitor of poly-γ-glutamic acid degradation, and a method for producing poly-γ-glutamic acid.

### Background Art

Lytic enzymes of Bacillus subtilis play key roles in life cycles, such as cell division, lysis of mother cells in the sporulation phase, germination of spores, and the like. Bacillus subtilis has different lytic enzymes that are specifically expressed in accordance with, for example, the process of cell division or sporulation phase. Many of such lytic enzymes have a binding domain for binding to a cell wall and an active domain to cause a cell wall to lyse. As lytic enzymes that express at the logarithmic growth phase of Bacillus subtilis, CwlB (see Kuroda, A. and J. Sekiguchi, 1991, "Molecular cloning and sequencing of a major Bacillus subtilis autolysin gene," J. Bacteriol. 173: 7304-7312 and Lazarevic, V., P. Margot, B. Sold, and D. Karamata, 1992, "Sequencing and analysis of the Bacillus subtilis lytRABC divergone: a regulatory unit encompassing the structural gene of the N-acetylmuramoyl-L-alanine amidase and its modifier," J. Gen. Microbiol. 138: 1949-1961) and CwlG (see Margot, P., C. Mauel, and D. Karamata, 1994, "The gene of the N-acetyl-glucosaminidase, a Bacillus subtilis cell wall hydrase not involved in vegetative cell autolysis," J. Bacteriol. 12: 535-545 and Rashid, M. H., M. Mori, and J. Sekiguchi, 1995. "Glucosaminidase of Bacillus subtilis: cloning, regulation, primary structure and biochemical characterization," Microbiology 141: 2391-2404) are known. As lytic enzymes that express at the final stage of cell division of Bacillus subtilis, CwlE (see Margot, P., M. Pagni, and D. Karamata, 1999, "Bacillus subtilis 168 gene lytF encodes a γ-D-glutamate-meso-diaminopimelate muropeptidase expressed by the alternative vegetative sigma factor, σD," Microbiology 145: 57-65 and Ohnishi, R., S. Ishikawa, and J Sekiguchi, 1999. "Peptidoglycan hydrase LytF plays a role in cell separation with LytE during vegetative growth of Bacillus subtilis," J. Bacteriol. 181: 3178-3184) and CwlF (see Ishikawa, S., Y. Hara, R. Ohnishi, and J. Sekiguchi, 1998, "Regulation of a new cell wall hydrase gene, CwlF, which affects cell separation in Bacillus subtilis," J. Bacteriol. 180: 2549-2555 and Margot, P., M. Wahlen, A. Gholamhuseinian, P. Piggot, and D. Karamata, 1998, "The lytE gene of Bacillus subtilis 168 encodes a cell wall hydrase," J. Bacteriol. 80: 749-752) are known.

Action mechanisms of lytic enzymes on the cell wall of the Bacillus subtilis, however, are not completely understood, and a method for inhibiting activity of the lytic enzymes has not yet been established. If activity of the lytic enzyme can be inhibited, microbial lysis can also be inhibited.

Poly-γ-glutamic acid (hereafter abbreviated to "PGA") is a polymer compound in which a carboxyl group at position γ is bound to an amino group at position α of glutamic acid via peptide bond. PGA is known as a sticky substance produced by Bacillus subtilis var. natto, and it has drawn attention as a novel polymeric material because of its various properties in recent years. From the viewpoint of various applications as a material, in particular, PGA of a relatively high molecular weight is preferable. However, a technique for producing PGA of a relatively high molecular weight with the use of a microorganism capable of producing PGA has not yet been known.

### Disclosure of the Invention

Under the above circumstances, the present invention intends to provide a lytic enzyme inhibitor that can regulate activity of a lytic enzyme for degrading a cell wall and a lysis inhibitor that can inhibit microbial lysis. The present invention is also intended to provide a method for producing PGA of a relatively high molecular weight and a method for inhibiting PGA degradation.

In order to attain the above objects, the present inventors have conducted concentrated studies. As a result, they discovered that a given gene product whose functions are unknown would inhibit activity of a lytic enzyme for degrading a cell wall and such gene product would inhibit activity of an enzyme that degrades PGA (i.e., decrease molecular weight). This has led to the completion of the present invention.

The lytic enzyme inhibitor, the lysis inhibitor, and the inhibitor of PGA degradation of the present invention are composed mainly of the Bacillus subtilis-derived YoeB gene or the YoeB protein encoded by a gene homologous to the YoeB gene.

The term "YoeB protein" used herein can be defined as any of proteins (a) to (c) below:
(a) a protein consisting of the amino acid sequence as shown in SEQ ID NO: 2;
(b) a protein consisting of an amino acid sequence derived from the amino acid sequence as shown in SEQ ID NO: 2 by substitution, deletion, addition, or insertion of 1 or several amino acids and having activity of binding to the Bacillus subtilis-derived lytic enzyme to inhibit lytic activity thereof; and
(c) a protein consisting of an amino acid sequence having 70% or higher homology to the amino acid sequence as shown in SEQ ID NO: 2 and having activity of binding to the Bacillus subtilis-derived lytic enzyme to inhibit lytic activity thereof.

The lytic enzyme inhibitor of the present invention can particularly inhibit lysis caused by the CwlE protein, CwlF protein, CwlS protein, CwlO protein, or YddH protein.

The "CwlE protein" can be defined as any of proteins (a) to (c) below:
(a) a protein consisting of the amino acid sequence as shown in SEQ ID NO: 4;
(b) a protein consisting of an amino acid sequence derived from the amino acid sequence as shown in SEQ ID NO: 4 by substitution, deletion, addition, or insertion of one or several amino acids and having activity of lysing the cell wall of Bacillus subtilis; and
(c) a protein consisting of an amino acid sequence having 70% or higher homology to the amino acid sequence as shown in SEQ ID NO: 4 and having activity of lysing the cell wall of Bacillus subtilis.

The "CwlF protein" can be defined as any of proteins (a) to (c) below:
(a) a protein consisting of the amino acid sequence as shown in SEQ ID NO: 6;
(b) a protein consisting of an amino acid sequence derived from the amino acid sequence as shown in SEQ ID NO: 6 by substitution, deletion, addition, or insertion of one or several amino acids and having activity of lysing the cell wall of Bacillus subtilis; and
(c) a protein consisting of an amino acid sequence having 70% or higher homology to the amino acid sequence as shown in SEQ ID NO: 6 and having activity of lysing the cell wall of Bacillus subtilis.

Further, the lysis inhibitor of the present invention can inhibit lysis of Bacillus subtilis, in particular. Bacillus subtilis strains are not limited to wild-type Bacillus subtilis strains, and an example thereof is a mutant lacking at least one type of secretory protease.

The "CwlS protein" can be defined as any of proteins (a) to (c) below:
(a) a protein consisting of the amino acid sequence as shown in SEQ ID NO: 55;
(b) a protein consisting of an amino acid sequence derived from the amino acid sequence as shown in SEQ ID NO: 55 by substitution, deletion, addition, or insertion of one or several amino acids and having activity of lysing the cell wall of Bacillus subtilis; and
(c) a protein consisting of an amino acid sequence having 70% or higher homology to the amino acid sequence as shown in SEQ ID NO: 55 and having activity of lysing the cell wall of Bacillus subtilis.

The "CwlO protein" can be defined as any of proteins (a) to (c) below:
(a) a protein consisting of the amino acid sequence as shown in SEQ ID NO: 57;
(b) a protein consisting of an amino acid sequence derived from the amino acid sequence as shown in SEQ ID NO: 57 by substitution, deletion, addition, or insertion of one or several amino acids and having activity of lysing the cell wall of Bacillus subtilis; and
(c) a protein consisting of an amino acid sequence having 70% or higher homology to the amino acid sequence as shown in SEQ ID NO: 57 and having activity of lysing the cell wall of Bacillus subtilis.

The "YddH protein" can be defined as any of proteins (a) to (c) below:
(a) a protein consisting of the amino acid sequence as shown in SEQ ID NO: 59;
(b) a protein consisting of an amino acid sequence derived from the amino acid sequence as shown in SEQ ID NO: 59 by substitution, deletion, addition, or insertion of one or several amino acids and having activity of lysing the cell wall of Bacillus subtilis; and
(c) a protein consisting of an amino acid sequence having 70% or higher homology to the amino acid sequence as shown in SEQ ID NO: 59 and having activity of lysing the cell wall of Bacillus subtilis.

The inhibitor of PGA degradation of the present invention can inhibit degradation of poly-γ-glutamic acid caused by the CwlE protein, CwlF protein, CwlS protein, or CwlO protein, among the above lytic enzymes. Among the above lytic enzymes, the YddH protein would not exhibit activity of PGA degradation, unlike the CwlE, CwlF, CwlS, and CwlO proteins.

Further, a microorganism of the present invention expresses the Bacillus subtilis-derived YoeB gene or a gene homologous to the YoeB gene downstream of the expression-inducible promoter so that lysis of the microorganism has been inhibited. The microorganism of the present invention particularly preferably lacks at least one type of secretory protease.

Also, the microorganism of the present invention is preferably capable of producing PGA. The microorganism of the present invention can inhibit activity of the lytic enzyme to degrade PGA by the YoeB protein. Examples of lytic enzymes that inhibit PGA degrading activity include the Bacillus subtilis-derived CwlE, CwlF, CwlS, and CwlO proteins and proteins functionally equivalent to the aforementioned proteins.

The method for producing PGA of the present invention comprises culturing microorganisms capable of producing PGA in the presence of the Bacillus subtilis-derived YoeB gene or the YoeB protein encoded by a gene homologous to the YoeB gene and recovering PGA produced in the medium. The method for producing PGA of the present invention may involve the use of microorganisms that express the Bacillus subtilis-derived YoeB gene or a gene homologous to the YoeB gene downstream of the expression-inducible promoter. Alternatively, the method may involve the addition of the YoeB protein to the medium. According to the method for producing PGA of the present invention, the above-mentioned microorganisms produce PGA while activity of PGA degradation of the PGA degrading enzyme is inhibited by the YoeB protein.

This description includes part or all of the contents as disclosed in the description and/or drawings of Japanese Patent Application No. 2006-281768, which is a priority document of the present application.

### Brief Description of the Drawings

Fig. 1(A) is a photograph showing the results of SDS-PAGE analysis of the outer surface protein purified with the anti-FLAG antibody extracted from the WE1YoeB3FL strain (lane 1) and the outer surface protein purified with the anti-FLAG antibody extracted from the WE1E3FL strain (lane 2). Fig. 1(B) is a photograph showing the results of SDS-PAGE analysis of the outer surface protein purified with the anti-FLAG antibody extracted from the WE1YoeB3FL strain (lane 1) and the results of zymography involving the addition of a cell-wall-component as a substrate (lane 2).
Fig. 2(A) is a photograph showing the results of SDS-PAGE analysis of the outer surface protein purified with the anti-FLAG antibody extracted from the WE1E3FL strain (lane 1) and of the outer surface protein purified with the anti-FLAG antibody extracted from the WE15xLysM3FL strain (lane 2). Fig. 2(B) is a photograph showing the results of SDS-PAGE analysis of the outer surface protein purified with the anti-FLAG antibody extracted from the WE1F3FL strain (lane 1) and of the outer surface protein purified with the anti-FLAG antibody extracted from the WE13xLysM3FL strain (lane 2).
Fig. 3 is a characteristic diagram showing the correlation between the reaction time and decrease in turbidity, when using a cell wall component as a substrate, of a sample to which His-CwlECTD has been added, a sample to which His-CwlECTD and His-YoeB have been added at 1:1, and a sample to which His-CwlECTD and His-YoeB have been added at 1:2.
Fig. 4 is a characteristic diagram showing the correlation between the reaction time and decrease in turbidity, when using a cell wall component as a substrate, of a sample to which the h-ΔCwlS protein has been added and a sample to which the h-ΔCwlS protein and the His-YoeB have been added at 1:2.
Fig. 5 is a characteristic diagram showing the correlation between the reaction time and decrease in turbidity, when using a cell wall component as a substrate, of a sample to which the 6His-C-CwlO protein has been added and a sample to which the 6His-C-CwlO protein and the His-YoeB have been added at 1:2.
Fig. 6 is a characteristic diagram showing the correlation between the reaction time and decrease in turbidity, when using a cell wall component as a substrate, of a sample to which the 6His-C-YddH protein has been added and a sample to which the 6His-C-YddH protein and His-YoeB have been added at 1:2.
Fig. 7 is a characteristic diagram showing the effects of lysis inhibition in the secretory protease-deficient Dpr8 strain, when YoeB proteins are overexpressed.
Fig. 8 is a photograph showing the results of SDS-polyacrylamide electrophoresis representing the results of comparison and examination of activity of the CwlE protein for PGA degradation in the presence of and in the absence of the YoeB protein.
Fig. 9 is a photograph showing the results of SDS-polyacrylamide electrophoresis representing the results of comparison and examination of activity of the CwlF protein for PGA degradation in the presence of and in the absence of the YoeB protein.
Fig. 10 is a photograph showing the results of SDS-polyacrylamide electrophoresis representing the results of comparison and examination of activity of the CwlS protein for PGA degradation in the presence of and in the absence of the YoeB protein.
Fig. 11 is a photograph showing the results of SDS-polyacrylamide electrophoresis representing the results of comparison and examination of activity of the CwlO protein for PGA degradation in the presence of and in the absence of the YoeB protein.
Fig. 12 is a photograph showing the results of SDS-polyacrylamide electrophoresis representing the results of comparison and examination of activity of the YwtD protein for PGA degradation in the presence of and in the absence of the YoeB protein.

### Best Modes for Carrying out the Invention

Whereafter, the present invention is described in detail with reference to the drawings.

The lytic enzyme inhibitor, the lysis inhibitor, and the inhibitor of PGA degradation of the present invention are composed mainly of the Bacillus subtilis YoeB gene or the YoeB protein encoded by a gene homologous to the YoeB gene.

An example of the nucleotide sequence of the Bacillus subtilis YoeB gene is shown in SEQ ID NO: 1, and the amino acid sequence of the YoeB protein encoded by the nucleotide sequence as shown in SEQ ID NO: 1 is shown in SEQ ID NO: 2. The YoeB protein encoded by the Bacillus subtilis YoeB gene is not limited to a polypeptide consisting of the amino acid sequence as shown in SEQ ID NO: 2. It may be a polypeptide consisting of an amino acid sequence derived from the amino acid sequence as shown in SEQ ID NO: 2 by substitution, deletion, addition, or insertion of one or a plurality of amino acids, which has activity of inhibiting the lytic activity of the lytic enzyme (hereafter referred to as a "mutant polypeptide"). The number of amino acids to be substituted, deleted, added, or inserted can be, for example, 1 to 20, preferably 1 to 10, and more preferably 1 to 5. A region in which one or a plurality of amino acids are substituted, deleted, added, or inserted is, for example, a region excluding a region between positions 24 and 181 of the amino acid sequence as shown in SEQ ID NO: 2. The region between positions 24 and 181 is considered to bind to a lytic enzyme. Accordingly, activity of inhibiting lytic activity of the lytic enzyme can be retained, even if one or a plurality of amino acids are substituted, deleted, added, or inserted in a region excluding the above region.

The YoeB protein encoded by the Bacillus subtilis YoeB gene is not limited to a polypeptide consisting of an amino acid sequence as shown in SEQ ID NO: 2. It may be a polypeptide having 70% or higher, preferably 80% or higher, more preferably 90% or higher, and most preferably 95% or higher homology to the amino acid sequence as shown in SEQ ID NO: 2, which has activity of binding to the Bacillus subtilis-derived lytic enzyme to inhibit lytic activity thereof (hereafter, it is also referred to as a "mutant polypeptide"). A homology value is a value determined by default using software that computes homology among a plurality of amino acid sequences (e.g., DANASYS or BLAST).

The Bacillus subtilis YoeB gene is not limited to a polynucleotide consisting of the nucleotide sequence as shown in SEQ ID NO: 1. It may be a polynucleotide that hybridizes under stringent conditions to a complementary strand of the polynucleotide consisting of the nucleotide sequence as shown in SEQ ID NO: 1, which encodes a polypeptide having activity of inhibiting lytic activity of the lytic enzyme (hereafter, it is referred to as a "mutant polynucleotide"). A mutant polynucleotide encodes a polypeptide consisting of an amino acid sequence different from the amino acid sequence as shown in SEQ ID NO: 2, which has activity of inhibiting lytic activity of the lytic enzyme (hereafter, it is referred to as a "mutant polypeptide").

Stringent conditions refer to conditions that so-called specific hybrids are formed, but non-specific hybrids are not formed. Under stringent conditions, for example, highly homologous DNAs hybridize to each other (e.g., homology therebetween is 50% or higher), and DNAs less homologous to each other would not hybridize. More specifically, as a stringent condition, hybridization takes place at 60°C and a salt concentration equivalent to 1× SSC, 0.1% SDS, and preferably 0.1× SSC, 0.1% SDS, which are washing conditions for general Southern hybridization. A mutant polynucleotide can be obtained by introducing a given mutation into a wild type YoeB gene derived from Bacillus subtilis. A method for introducing mutation is not particularly limited, and so-called site-directed mutagenesis can be employed.

Whether or not the various above-mentioned mutant polypeptides have the activity of inhibiting the lytic activity of the lytic enzyme can be determined in the following manner. Specifically, a mutant containing a gene encoding a mutant polypeptide substituted with a wild-type YoeB gene is prepared. Alternatively, a mutant where a wild-type YoeB gene is disrupted and a mutant polypeptide is introduced is prepared. Such mutant is cultured and morphologically observed. When a mutant polypeptide has activity of inhibiting lytic activity of the lytic enzyme, the shape of the mutant is rounder than that of a wild-type strain. When a mutant polypeptide does not have activity of inhibiting lytic activity of the lytic enzyme, the shape of the mutant becomes rod-like, as with a wild-type strain. Thus, morphological observation of the mutant expressing a gene that encodes a mutant polypeptide enables determination of whether or not the mutant polypeptide has the activity of inhibiting lytic activity of the lytic enzyme.

The main component of the lytic enzyme inhibitor, the lysis inhibitor, or the inhibitor of PGA degradation of the present invention is not limited to a protein encoded by the Bacillus subtilis-derived YoeB gene. The main component may be the YoeB protein encoded by a homologous gene corresponding to the Bacillus subtilis YoeB gene, which has been isolated from a microorganism other than Bacillus subtilis. An example of a microorganism other than Bacillus subtilis is Bacillus licheniformis.

Hereafter, a method for isolating a homologous gene of the YoeB gene from a microorganism other than Bacillus subtilis is described. However, a person skilled in the art can isolate a target homologous gene in accordance with a conventional technique, without being limited to the technique described below.

At the outset, a database storing nucleotide sequence information is used to identify a highly homologous gene using the nucleotide sequence as shown in SEQ ID NO: 1 as the query sequence. Subsequently, primers for specifically amplifying the identified gene are designed and chemically synthesized. Subsequently, genome DNA is extracted from the target microorganism in accordance with a conventional technique. The target homologous gene of the YoeB gene can be amplified via PCR using the extracted genome DNA as a template and the above primers, and then be isolated.

Whether or not the isolated homologous gene of the YoeB gene has functions equivalent to those of the YoeB protein can be examined by preparing an expression vector into which the isolated homologous gene of the YoeB gene has been incorporated, transforming the same into the target microorganism, and observing the shape when the transformed microorganism is cultured under conditions in which the above homologous gene is expressed. When the isolated homologous gene of the YoeB gene has functions equivalent to those of the YoeB protein, the shape of the transformed microorganism becomes rounder than that of the wild-type strain. When the isolated homologous gene of the YoeB gene has functions similar to those of the YoeB protein, the shape of the transformed microorganism would be similar to that of the wild-type strain.

The Bacillus subtilis YoeB gene and a gene homologous to the YoeB gene can be used to prepare the YoeB protein, which is a main component of the lytic enzyme inhibitor and the lysis inhibitor of the present invention. The YoeB protein may be produced with the use of the above Bacillus subtilis YoeB gene or a gene homologous to the YoeB gene by using a recombinant vector, which is prepared by incorporating the gene of interest into a vector that can replicate and maintain the gene of interest in a host cell, can stably express the enzyme, and can stably maintain the gene, thereby transforming the host microorganism.

When an E. coli host is used, a vector is not particularly limited, and pUC118, pBR322, pHY300PLK (Yakult Honsha Co., Ltd.), and other vectors can be used. When a Bacillus subtilis host is used, pUB110, pHSP64 (Sumitomo et al., Biosci. Biotechnol, Biocem., 59, 2172-2175, 1995), pHY300PLK, or other vectors can be used, without particular limitation.

Host cells can be transformed via a protoplast method, a competent cell method, electroporation, or other techniques. A host cell is not particularly limited, and examples include Gram-positive strains such as Bacillus subtilis, Gram-negative strains such as E. coli, Actinomycetes such as Streptomyces, yeast such as Saccharomyces, and fungi such as Aspergillus.

The resulting transformants may be cultured under adequate conditions using a medium containing carbon sources, nitrogen sources, metal salts, vitamins, and the like assimilable by microorganisms. The resulting culture solution may be subjected to enzyme fractionation or purification in accordance with conventional techniques to obtain YoeB proteins having activity of inhibiting lytic activity of the lytic enzyme via concentration via ultrafiltration, lyophilization, spray drying, crystallization, or the like.

When the lytic enzyme inhibitor and the lysis inhibitor of the present invention are added to a medium in which microorganisms are cultured or to a buffer in which microorganisms are suspended, activity of lytic enzymes expressed by the microorganisms can be lowered, and lysis of such microorganisms can be inhibited. Lytic enzymes that are inhibited by the lytic enzyme inhibitor of the present invention are not particularly limited, and examples thereof include a lytic enzyme that is expressed in a cell-cycle-dependent manner and a lytic enzyme that is expressed in a cell-cycle-independent manner. In particular, the lytic enzyme inhibitor of the present invention can inhibit lysis caused by the CwlE, CwlF, CwlS, CwlO, and YddH proteins, which are lytic enzymes of Bacillus subtilis. The CwlE, CwlF, CwlS, CwlO, and YddH proteins are known to be lytic enzymes that are expressed at the cell-division surface and at both poles of the cell at the final stage of Bacillus subtilis cell division. The CwlE, CwlF, CwlS, CwlO, and YddH proteins each contain a signal peptide, a cell-wall-binding domain, and a active domain for cell wall lysis (i.e., a D,L-endopeptidase domain) in that order from the N-terminus. The lytic enzyme inhibitor and the lysis inhibitor of the present invention bind to the active domain for cell wall lysis of the CwlE, CwlF, CwlS, CwlO, and YddH proteins and inhibit cell wall lytic activity of the CwlE, CwlF, CwlS, CwlO, and YddH proteins to inhibit lysis.

When the inhibitor of PGA degradation of the present invention is added at the time of culture of microorganisms capable of producing PGA, PGA degrading activity of lytic enzymes having activity of PGA degradation can be lowered, and decrease of molecular weights of PGA produced by the microorganisms can be prevented. Examples of lytic enzymes to be inhibited by the inhibitor of PGA degradation of the present invention include CwlE, CwlF, CwlS, and CwlO proteins. PGA with a relatively high molecular weight can be produced by lowering PGA degrading activity of lytic enzymes.

The nucleotide sequence of the gene encoding the CwlE protein (the CwlE gene) is shown in SEQ ID NO: 3, and the amino acid sequence of the CwlE protein is shown in SEQ ID NO: 4. The nucleotide sequence of the gene encoding the CwlF protein (the CwlF gene) is shown in SEQ ID NO: 5, and the amino acid sequence of the CwlF protein is shown in SEQ ID NO: 6. The nucleotide sequence of the gene encoding the CwlS protein (the CwlS gene) is shown in SEQ ID NO: 54, and the amino acid sequence of the CwlS protein is shown in SEQ ID NO: 55. The nucleotide sequence of the gene encoding the CwlO protein (the CwlO gene) is shown in SEQ ID NO: 56, and the amino acid sequence of the CwlO protein is shown in SEQ ID NO: 57. The nucleotide sequence of the gene encoding the YddH protein (the YddH gene) is shown in SEQ ID NO: 58, and the amino acid sequence of the YddH protein is shown in SEQ ID NO: 59.

The term "protein that is functionally equivalent to the Bacillus subtilis-derived CwlE, CwlF, CwlS, CwlO, or YddH protein" refers to, for example, a protein consisting of an amino acid sequence derived from the amino acid sequence as shown in SEQ ID NO: 4, 6, 55, 57, or 59 by substitution, deletion, addition, or insertion of 1 or several amino acids and having activity of lysing the cell wall of Bacillus subtilis or a protein consisting of an amino acid sequence having 70% or higher, 80% or higher, 90% or higher, more preferably 95% or higher, further preferably 96% or higher, particularly preferably 97% or higher, more particularly preferably 98% or higher, and most preferably 99% or higher homology to the amino acid sequence as shown in SEQ ID NO: 4, 6, 55, 57, or 59 and having activity of lysing the cell wall of Bacillus subtilis. The lytic enzyme inhibitor of the present invention can act on such proteins.

The number of amino acids to be substituted, deleted, added, or inserted can be, for example, 1 to 40, preferably 1 to 20, and more preferably 1 to 10. A region in which one or a plurality of amino acids are substituted, deleted, added, or inserted is, for example, a region excluding a region between positions 371 and 488 of the amino acid sequence as shown in SEQ ID NO: 4, a region between positions 218 and 334 of the amino acid sequence as shown in SEQ ID NO: 6, a region between positions 1 and 290 of the amino acid sequence as shown in SEQ ID NO: 55, a region between positions 1 and 343 of the amino acid sequence as shown in SEQ ID NO: 57, and a region between positions 1 and 206 of the amino acid sequence as shown in SEQ ID NO: 59. The region between positions 371 and 488 of the amino acid sequence as shown in SEQ ID NO: 4, the region between positions 218 and 334 of the amino acid sequence as shown in SEQ ID NO: 6, the region between positions 1 and 290 of the amino acid sequence as shown in SEQ ID NO: 55, the region between positions 1 and 343 of the amino acid sequence as shown in SEQ ID NO: 57, and the region between positions 1 and 260 of the amino acid sequence as shown in SEQ ID NO: 59 are considered to be regions to which the lytic enzyme inhibitor of the present invention binds. Accordingly, activity of the lytic enzyme inhibitor of the present invention can be retained, even if one or a plurality of amino acids are substituted, deleted, added, or inserted in a region excluding the above regions.

As described above, the lytic enzyme inhibitor and the lysis inhibitor of the present invention enable inhibition of cell wall lysing activity of the lytic enzyme via binding of the YoeB protein to the active domain of the lytic enzyme for cell wall lysis, such as the CwlE, CwlF, CwlS, CwlO, or YddH protein. This inhibits lysis of the microorganisms that express the above lytic enzymes and enables culture for a relatively long period of time. When a material is produced with the use of a microorganism such as Bacillus subtilis, for example, the amount of material production can be increased if each cell can be cultured for a longer period of time. With the addition of the lytic enzyme inhibitor of the present invention when culturing microorganisms that produce materials of interest, the efficiency of production of such materials can be improved.

The lysis inhibitor of the present invention can be applied to a wide range of microorganisms. Application thereof to bacteria of Bacillus, such as Bacillus subtilis, is particularly preferable. The target microorganisms of the lysis inhibitor of the present invention are not limited to wild-type microorganisms. For example, mutants having a variety of mutations can also be targets. An example of a target microorganism of the lysis inhibitor of the present invention is a mutant that lacks at least one type of secretory protease. Since such mutant lacks secretory protease, it is preferable as a host that produces a material, such as a useful protein, to the outside of the cell. A system that produces the material of interest with the use of such mutant can inhibit the lysis of the mutant through the addition of the lysis inhibitor of the present invention. This enables culture of the mutant for a relatively long period of time. Consequently, the efficiency of the production of such materials can be improved, and the cost of material production can be reduced.

The amount of the lytic enzyme inhibitor or the lysis inhibitor of the present invention to be added is not particularly limited. Such amount can adequately be determined, provided that such inhibitors can bind to the active domain of the lytic enzyme for cell wall lysis, such as the CwlE, CwlF, CwlS, CwlO, or YddH protein.

As described above, the inhibitor of PGA degradation of the present invention enables inhibition of PGA degrading activity of the lytic enzyme via binding of the YoeB protein to the active domain of the lytic enzyme having activity of PGA degradation for cell wall lysis, such as the CwlE, CwlF, CwlS, or CwlO protein. When microorganisms having the capacity for PGA production and expressing the lytic enzyme are cultured in the presence of the inhibitor of PGA degradation, accordingly, PGA of a relatively high molecular weight can be produced. The term "PGA of a relatively high molecular weight" refers to PGA having a molecular weight that is significantly higher than the average molecular weight of PGA produced by the microorganisms in the absence of the inhibitor of PGA degradation of the present invention. Since the inhibitor of PGA degradation of the present invention can inhibit microbial lysis as described above, PGA of a relatively high molecular weight can be produced for a long period of time.

The inhibitor of PGA degradation of the present invention can be applied to a wide range of microorganisms capable of PGA production. Examples of microorganisms capable of PGA production include Bacillus subtilis var. natto, Bacillus licheniformis, Bacillus megaterium, Bacillus anthracis, Bacillus halodurans, and Natrialba aegyptiaca, Hydra.

The present invention can provide microorganisms that are modified so as to express the lytic enzyme inhibitor or the gene encoding the lysis inhibitor under the control of an expression-inducible promoter. By culturing the microorganism of the present invention under the expression-inducible conditions, the lysis inhibitor is secreted to the medium, which in turn enables inhibition of the lysis for a relatively long period of time.

Accordingly, the microorganism of the present invention having such constitution may be utilized to produce materials, such as useful proteins, to enhance the efficiency of the production of such materials. The microorganism of the present invention is particularly preferably modified to express the lytic enzyme inhibitor or the gene encoding the lysis inhibitor downstream of the expression-inducible promoter with the use of a mutant that lacks at least one type of secretory protease as a host. Such constitution enables prevention of degradation of target substances, such as useful proteins, which in turn allows realization of higher production efficiency.

The term "expression-inducible promoter" used herein refers to a promoter that has a function of enhancing expression of the gene located downstream in the presence of a given material. An expression-inducible promoter used for the microorganism of the present invention is not particularly limited. An example is a promoter (P_{spac}) that is regulated by a lac operon repressor, i.e., lacl, which induces expression of downstream genes in the presence of IPTG.

As the microorganism of the present invention, the microorganism capable of PGA production is particularly preferably modified so as to express the gene encoding the inhibitor of PGA degradation under the control of the expression-inducible promoter. Culture of such microorganisms in a medium enables production of PGA of a relatively high molecular weight. When such microorganisms are cultured in a medium to produce PGA of a high molecular weight, separately prepared inhibitor of PGA degradation may be added to the medium.

Hereafter, the present invention is described in greater detail with reference to the examples, although the technical scope of the present invention is not limited thereto.

### [Example 1] Examination of YoeB protein-lytic enzyme interaction

In this example, the interaction between the YoeB protein (SEQ ID NO: 2) encoded by the YoeB gene (SEQ ID NO: 1) and the CwlE protein encoded by the CwlE gene (SEQ ID NO: 3) was examined.

Specifically, the WE1YoeB3FL strain that expresses the YoeB-3×FLAG protein induced with IPTG and the WE1E3FL strain that expresses the CwlE-3×FLAG protein induced with IPTG were first constructed. The WE1YoeB3FL strain and the WE1E3FL strain were prepared using the WE1 strain (Yamamoto, H. et al., 2003 "Localization of the vegetative cell wall hydrases LytC, LytE, LytF on the Bacillus subtilis cell surface and stability of these enzymes to cell wall-bound or extracellular proteases," J. Becteriol., 185: 6666-6677). The WE1 strain has a genotype in which the minor extracellular serine protease (i.e., the epr gene) is disrupted via insertion of a tetracycline-resistant gene and the cell wall associate protease (i.e., the wprA gene) is disrupted via insertion of a kanamycin-resistant gene.

The WE1YoeB3FL strain was prepared by transforming the pCA3FLYoeB vector containing the gene encoding the YoeB-3×FLAG protein downstream of the IPTG-inducible promoter into the WE1 strain. The WE1E3FL strain was prepared by transforming the pCA3FLCE vector containing the gene encoding the CwlE-3×FLAG protein downstream of the IPTG-inducible promoter into the WE1 strain. A 3×FLAG-fused protein can be detected using the anti-FLAG M2 monoclonal antibody (Sigma).

Subsequently, the outer surface protein was extracted from the constructed WEYoeB3FL and WE1E3FL strains, respectively. Specifically, these strains were each inoculated in LB agar medium and subjected to pre-pre-culture at 37°C for 12 hours. The resulting single colony was inoculated in 5 ml of LB liquid medium and then subjected to pre-culture at 30°C for 10 hours. After the pre-culture, the pre-cultured strain was inoculated in 50 ml of LB liquid medium to bring the OD₆₀₀ value to 0.01, and main culture was conducted until the OD₆₀₀ value reached 2.0. After the completion of the culture, the culture solution was centrifuged at 10,000 rpm for 10 minutes. The supernatant was removed and the precipitate was washed with 25 mM Tris-HCl (pH 7.2). The washed precipitate was suspended in 3M LiCl (25 mM Tris-HCl, pH 7.2) to a density of 10 µl/OD, and the suspension was allowed to stand on ice for 20 minutes. Thereafter, centrifugation was carried out at 4°C and 10,000 rpm for 10 minutes. The supernatant was recovered, TCA (trichloroacetic acid) was added to a final concentration of 5%, the mixture was thoroughly mixed, and the resulting suspension was allowed to stand on ice for 60 minutes. Thereafter, centrifugation was carried out at 4°C and 15,000 rpm for 5 minutes. The supernatant was removed, and the precipitate was rinsed with 70% ethanol to thoroughly remove TCA. Thus, outer surface proteins were extracted from the WE1YoeB3FL strain and the WE1E3FL strain, respectively.

Subsequently, 200 µl of affinity gel (Anti-FLAG M2 Affinity Gel Freezer-Safe (Sigma)) substituted with TBS buffer (50 mM Tris-HCl, 150 mM NaCl (pH 7.4)) was added and mixed. The mixture was mildly (8 rpm) agitated at 4°C for 1 hour. Thereafter, centrifugation was carried out at 5,000 rpm for 1 minute. The supernatant was removed while carefully refraining from suctioning the precipitated gel. Thereafter, 500 µl of TBS buffer was added to prepare a suspension. Thereafter, the gel was transferred to the Spin Column (Micro Bio Spin Chromatography Columns (BIO RAD)), and the gel was trapped via centrifugation at 1,000 g for 30 seconds. Thereafter, the resultant was washed three times with 500 µl of TBS buffer. Subsequently, 100 µl of Glycine HCl elution buffer (0.1M glycine-HCl) was sequentially added thereto and thoroughly agitated. Elution was carried out 1 minute later at 1,000 g for 30 seconds, and this procedure was repeated two times. The products were combined to prepare an eluted solution.

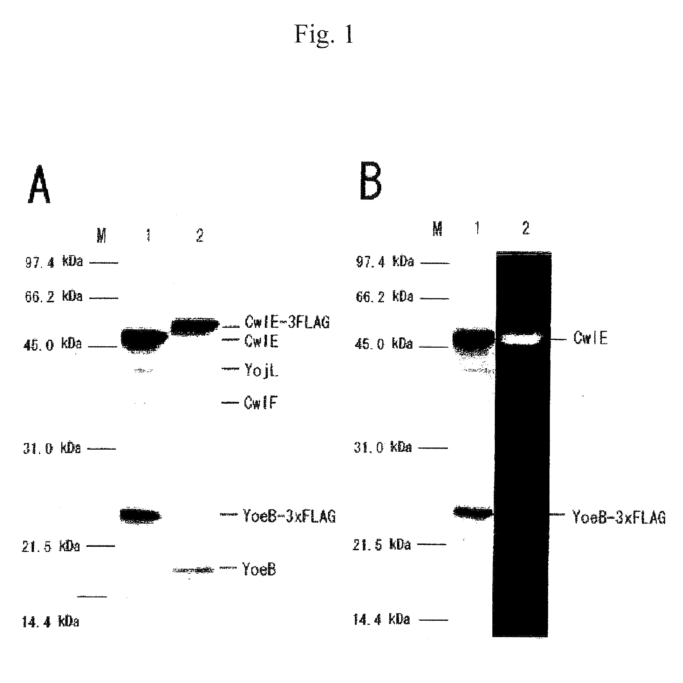

Subsequently, the band pattern of the resulting eluted solution was inspected via SDS-PAGE. Electrophoresis was carried out by charging proteins in amounts corresponding to the amount extracted from the cells (OD₆₀₀ = 200) to the relevant lanes. The results are shown in Fig. 1A. In Fig. 1A, lane 1 represents an outer surface protein purified with the anti-FLAG antibody extracted from the WE1YoeB3FL strain and lane 2 represents an outer surface protein purified with the anti-FLAG antibody extracted from the WE1E3FL strain.

As is apparent from lane 1 of Fig. 1A, a band was detected at positions corresponding to the CwlE protein, the CwlF protein, and the YojL protein in the outer surface protein purified with the anti-FLAG antibody extracted from the WE1YoeB3FL strain. The integrated values and the proportions thereof are shown in Table 1.

**Table 1**

| | Integrated value | Molecular weight (Da) | Integrated value/Molecular weight | Proportion relative to YoeB (1) |
|---|---|---|---|---|
| CwlE | 30057 | 51229 | 0.59 | 0.89 |
| YojL | 989 | 44073 | 0.02 | 0.03 |
| CwlF | 1247 | 37162 | 0.03 | 0.05 |
| YoeB | 13201 | 19984 | 0.66 | 1.00 |

The results demonstrated that the YoeB protein were bound to the CwlE, CwlF, and YojL proteins when OD₆₀₀ of the cells was 2.0. The YojL protein is a component known as peptidoglycan hydrolase (the DL-endopeptidase II family). As is apparent from lane 2 of Fig. 1A, a band was detected at the position corresponding to the YoeB protein in the outer surface protein purified with the anti-FLAG antibody extracted from the WE1E3FL strain.

In order to verify that a protein interacting with the YoeB protein was the CwlE protein, zymography was carried out using a cell wall component as a substrate. The cell wall component was prepared in the following manner. Specifically, Bacillus subtilis 168 strain was first cultured in a 4-liter system, harvested, suspended in a 4M LiCl solution, and then boiled for 15 minutes. The solution was cooled to room temperature, glass beads (ϕ 0.1mm) were added thereto, and the strains were fragmented using a homogenizer for 1 hour, followed by fragmentation using ultrasound waves for 30 minutes. The resulting solution of fragmented strains was allowed to stand and the supernatant was recovered. The supernatant was recovered after centrifugation at 3,000 rpm for 5 minutes to completely remove glass beads. Subsequently, centrifugation was carried out at 13,000 rpm for 10 minutes to recover the cell wall component in the precipitated fraction. The precipitated fraction was then suspended in a 4% (w/v) SDS solution, and the resultant was boiled for 15 minutes. Then, the resultant was cooled to room temperature and then washed with ion-exchange water until SDS was completely removed. The resulting solution was designated as a solution of cell wall component. The density of the cell wall component in the solution was calculated to be 1.1mg/ml based on the assayed OD₅₄₀ value of 1.0.

Zymography was carried out in the following manner. At the outset, the cell wall component was added to a separation gel to a final density of 0.05% when performing SDS-PAGE. After the completion of electrophoresis, the gel was mildly shaken in Renaturation Buffer (25 mM Tris-HCl (pH 7.2), 1% Triton-X100) at 37°C for 1 to 16 hours. Thereafter, the resultant was soaked in a staining solution (0.01% methylene blue, 0.01% KOH), mildly shaken at room temperature, and then decolored with ion-exchange water until an active band became visually observable.

The results are shown in Fig. 1B. In Fig. 1B, lane 1 represents an outer surface protein purified with the anti-FLAG antibody extracted from the WE1YoeB3FL strain and lane 2 represents the results of zymography carried out with the use of a cell wall component as a substrate. As shown in lane 2 of Fig. 1B, a band of substantially the same intensity was detected at the same position as in the case of the CwlE protein as a protein that had interacted with the YoeB protein.

The results shown in Figs. 1A and 1B demonstrated that the YoeB protein would bind to the CwlE protein.

Subsequently, the domain of the CwlE protein to which the YoeB protein had bound was inspected. The CwlE protein comprises a signal peptide, a cell-wall-binding domain, and an active domain in that order from the N-terminus. Thus, the WE15xLysM3FL strain expressing a mutant CwlE-3×FLAG protein having no active domain and containing a signal peptide, a cell-wall-binding domain, and a FLAG tag in that order from the N-terminus induced with IPTG, was constructed.

The WE1E3FL strain and the WE15xLysM3FL strain mentioned above were used, the outer surface proteins were purified using the anti-FLAG antibody, and the band pattern was inspected via SDS-PAGE in the same manner as in the above experiment. Electrophoresis of the WE1E3FL strain was carried out by charging proteins in amounts corresponding to the amount extracted from the cells (OD₆₀₀ = 200) to the relevant lanes. Electrophoresis of the WE15xLysM3FL strain was carried out by charging proteins in amounts corresponding to the amount extracted from the cells (OD₆₀₀ = 2,000) to the relevant lanes.

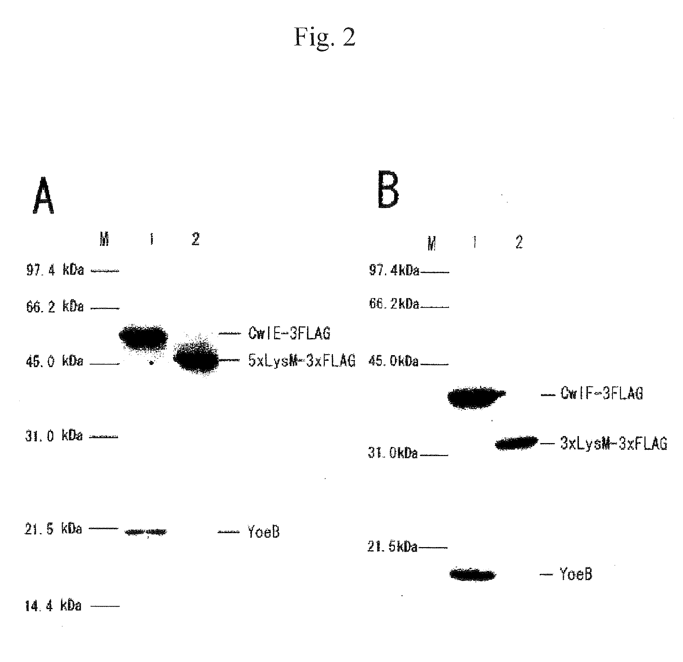

The results are shown in Fig. 2A. In Fig. 2A, lane 1 represents an outer surface protein purified with the anti-FLAG antibody extracted from the WE1E3FL strain and lane 2 represents an outer surface protein purified with the anti-FLAG antibody extracted from the WE15xLysM3FL strain. As is apparent from Fig. 2A, a band of the YoeB protein was detected in lane 1 but was not detected in lane 2. Such results demonstrated that the YoeB protein was bound to an active domain of the CwlE protein.

As shown in Fig. 1A, the YoeB protein was also found to have been bound to the CwlF protein. Thus, the site of the CwlF protein interacting with the YoeB protein was also inspected. The CwlF protein is a major cell wall degrading enzyme of Bacillus subtilis, and it is known to exhibit a localization pattern similar to that of the CwlE protein (i.e., a cell division surface and both poles).

In this experiment, the WE1F3FL strain that expresses the CwlF-3×FLAG protein induced with IPTG and the WE13xLysM3FL strain that expresses the mutant CwlF-3×FLAG protein having no an active domain and containing a signal peptide, a cell wall binding domain, and a FLAG tag in that order from the N-terminus induced with IPTG were constructed. The WE1F3FL strain and the WE13xLysM3FL strain mentioned above were used, the outer surface proteins were purified using the anti-FLAG antibody, and the band pattern was inspected via SDS-PAGE in the same manner as in the above experiment.

The results are shown in Fig. 2B. In Fig. 2B, lane 1 represents an outer surface protein purified with the anti-FLAG antibody extracted from the WE1F3FL strain and lane 2 represents an outer surface protein purified with the anti-FLAG antibody extracted from the WE13xLysM3FL strain. As is apparent from Fig. 2B, a band of the YoeB protein was detected in lane 1 but was not detected in lane 2. Such results demonstrated that the YoeB protein was also bound to an active domain of the CwlF protein as in the case of the CwlE protein.

### [Example 2] Effects of YoeB protein for inhibiting lytic enzyme activity 1

In this example, the way that the YoeB protein would act on the cell wall lysing activity of a lytic enzyme was inspected via activity assay in liquid. In this example, cell wall lysing activity of the C-terminal active domain (CwlECTD) of CwlE was assayed using the cell wall component prepared in the same manner as in Example 1 as a substrate in the presence of or in the absence of the YoeB protein.

In this example, cell wall lysing activity was assayed using 20 mM MES buffer (pH 6.5). The cell wall component in an amount corresponding to OD₅₄₀ = 0.6 was washed with the assay buffer, and the cell wall was precipitated via centrifugation. The supernatant was removed, 2 ml of assay buffer was added to the precipitate to prepare a suspension, a cell wall component was prepared to adjust the final OD₅₄₀ value to 0.3, and the resultant was designated as a substrate solution.

Activity assay was carried out by allowing the His-tag-fused C-terminal active domain of CwlE (His-CwlECTD) to act on a substrate solution and assaying the turbidity of the substrate solution. Specifically, the cell wall lysing activity of His-CwlECTD was evaluated in terms of decrease in turbidity. The turbidity of the substrate solution was assayed using the ultraviolet-visible spectrophotometer (V-560, JASCO Corporation). Decrease in turbidity was determined by designating the OD₅₄₀ value 0 minutes after the initiation of assay as 100% and calculating the decrease based on the OD₅₄₀ values 5, 10, 20, and 30 minutes thereafter.

In this example, a sample prepared by adding 2 nmol (29 µg) of His-CwlECTD to a substrate solution, a sample prepared by adding 2 nmol (29 µg) of His-CwlECTD and 2 nmol (38.1 µg) of His-YoeB to a substrate solution, and a sample prepared by adding 2 nmol (29 µg) of His-CwlECTD and 4 nmol (76.1 µg) of His-YoeB to a substrate solution were assayed.

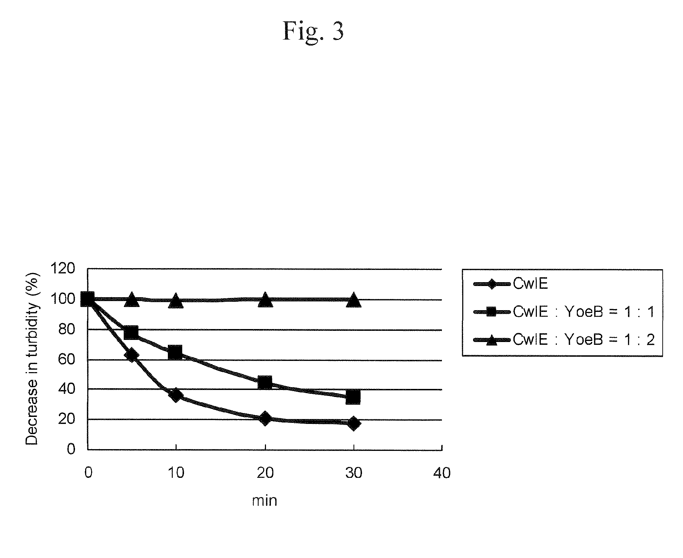

The correlation between the reaction time and decrease in turbidity is shown in Fig. 3. As is apparent from Fig. 3, the OD₅₄₀ value of the sample prepared by adding His-CwlECTD to the substrate solution began to significantly decrease immediately after the initiation of assay. Thus, His-CwlECTD was found to have strong lytic activity on the cell wall. In contrast, the sample prepared by adding the equivalent amount of His-CwlECTD and His-YoeB by mole to the substrate solution exhibited a decrease in the OD₅₄₀ value immediately after the initiation of assay; however, a milder curve was obtained than with a sample prepared by adding His-CwlECTD only. This indicates that activity of His-CwlECTD could be inhibited with the addition of the equivalent amount of His-YoeB by mole. In the case of a sample prepared by adding His-CwlECTD and His-YoeB at a ratio of 1:2 by mole, the OD₅₄₀ value did not substantially decrease immediately after the initiation of assay, and the value remained at a substantially constant level. This indicates that addition of His-YoeB in an amount twice the amount of His-CwlECTD would inhibit substantially 100% activity of His-CwlECTD by mole.

### [Example 3] Effects of YoeB protein for inhibiting lytic enzyme activity 2

In this example, cell wall lysing activity of the C-terminal active domains of CwlS, CwlO, and YddH was assayed using the cell wall component prepared in the same manner as in Example 1 as a substrate in the presence of or in the absence of the YoeB protein.

### <Preparation of C-terminal active domain of CwlS>

The 3'-terminal DNA sequence of the cwlS gene corresponding to the D,L-endopeptidase domain (including a region from nucleotide 290 to the termination codon and a 61-bp transcription terminator-like sequence) was amplified via PCR using the BF-YOJL primer (gcgcggatcctcaaacattcaaataggttcg: SEQ ID NO: 60) and the KR-YOJL primer (gcgcggtaccggtcaatcattgtctggta: SEQ ID NO: 61) and the genomic DNA extracted from the Bacillus subtilis 168 strain as a template. The underlined nucleotide sequences of the above BF-YOJL and KR-YOJL primers indicate the BamHI and KpnI restriction enzyme recognition sequences, respectively. After amplification via PCR, the resulting PCR product was digested with the BamHI and KpnI restriction enzymes to obtain an insert.

Subsequently, the pQE30 plasmid was digested with BamHI and KpnI, and the resultant was ligated to the above insert to construct the pQEΔojL plasmid. The pQE30 plasmid was developed for the purpose of expressing an His-tag fusion protein in E. coli.

Whether or not a DNA sequence of the insert had been properly inserted was verified via sequencing. The pQEΔojL plasmid, which was verified to have been properly inserted, was introduced into the E. coli JM109 strain by the competent method. The E. coli M13(pREP4) strain into which the pQEΔojL plasmid had been inserted is hereafter referred to as "M13 (pQEΔojL)."

M13 (pQEΔojL) was cultured in 200 ml of LB medium containing 100 µg/ml ampicillin, 25 µg/ml kanamycin, and 2% glucose at 37°C. When OD₆₀₀ of the culture solution was 1.0, IPTG (isopropyl 1-thio-β-D-galactoside) was added to the medium to a final concentration of 1 mM, and culture was conducted for an additional 5 hours. After culture, the culture product was centrifuged (8,000 rpm, 10 minutes, 4°C) to harvest cells, and the cells were suspended in the initiation buffer (5 ml; 1 × phosphate buffer (pH 7.0) containing 10 mM imidazole).

Subsequently, the resultant was subjected to ultrasonic fragmentation (on: 0.5 seconds; off: 1 second/on: 30 seconds; off: 30 seconds; 10 minutes in total) and then centrifugation (12,000 rpm, 10 minutes, 4°C). The resulting supernatant was filtered through a 0.45-µm filter. The h-ΔCwlS protein extracted via filtration was purified in accordance with a conventional technique of His-Trap column purification of GE Healthcare and Biosciences. The purified h-ΔCwlS protein solution was further dialyzed with 20 mM phosphate buffer (pH 7.0) to prepare the h-ΔCwlS protein solution.

### <Preparation of C-terminal active domain of CwlO>

The 3'-terminal DNA sequence of the cwlO gene corresponding to the D,L-endopeptidase domain (including a region from nucleotide 343 to the termination codon and an 84-bp transcription terminator-like sequence) was amplified via PCR using the BF-YVCE primer (gcgcggatccgaaggcgcgatcagcgtt: SEQ ID NO: 62) and the KR-YVCE primer (gcgcggtaccgctcaacacgattaaatgtat: SEQ ID NO: 63) and the genomic DNA extracted from the Bacillus subtilis 168 strain as a template. The underlined nucleotide sequences of the above BF-YVCE and KR-YVCE primers indicate the BamHI and KpnI restriction enzyme recognition sequences, respectively.

The resulting PCR product was used to prepare the pQEVCE plasmid in the same manner as with the above <Preparation of C-terminal active domain of CwlS>, and the E. coli M13 (pQEVCE) into which pQEVCE had been introduced was obtained. The obtained M13 (pQEVCE) was used to prepare the 6His-C-CwlO protein solution in the same manner as with the above <Preparation of C-terminal active domain of CwlS>.

### <Preparation of C-terminal active domain of YddH>

The 3'-terminal DNA sequence of the YddH gene corresponding to the D,L-endopeptidase domain (including a region from nucleotide 206 to the termination codon and a 9-bp untranslated region) was amplified via PCR using the BF-YDDH primer gcgcggatccgatttctatgaaacggtcatg: SEQ ID NO: 64) and the KR-YDDH primer (gcgcggtacccactcctagttatttaatgcg: SEQ ID NO: 65) and the genomic DNA extracted from the Bacillus subtilis 168 strain as a template. The underlined nucleotide sequences of the above BF-YDDH and KR-YDDH primers indicate the BamHI and KpnI restriction enzyme recognition sequences, respectively.

The resulting PCR product was used to prepare the pQEDDH plasmid in the same manner as with the above <Preparation of C-terminal active domain of CwlS>, and the E. coli M13 (pQEDDH) into which pQEDDH had been introduced was obtained. The obtained M13 (pQEDDH) was used to prepare the 6His-C-YddH protein solution in the same manner as with the above <Preparation of C-terminal active domain of CwlS>.

### <Assay of cell wall lysing activity and results thereof>

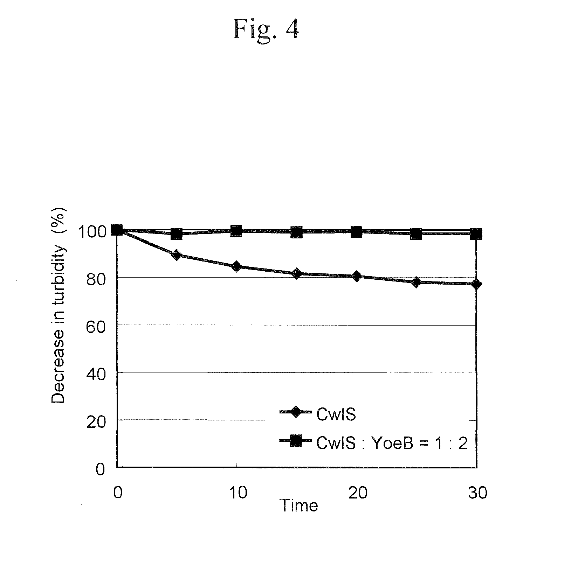

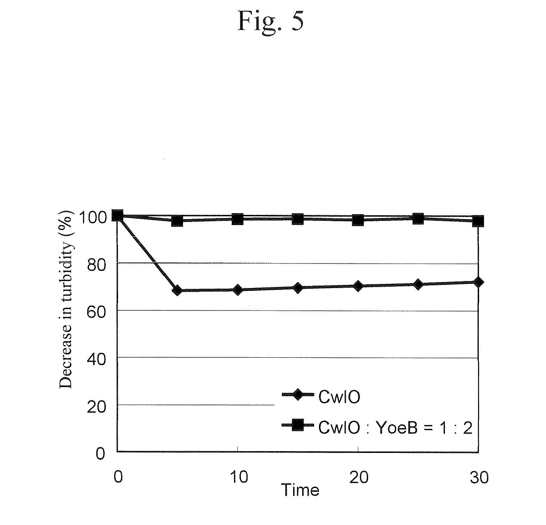

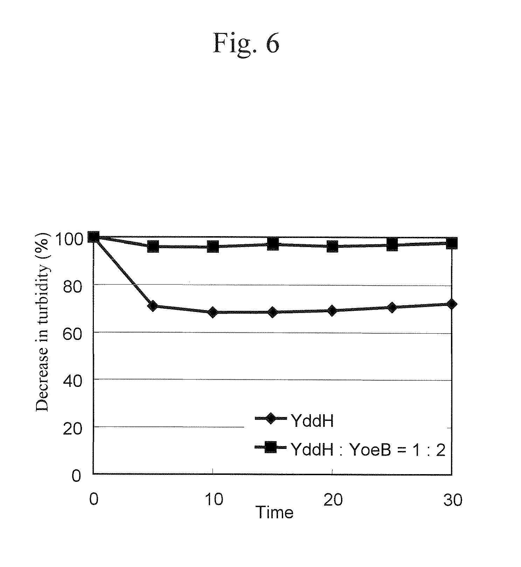

Cell wall degrading activity was assayed in accordance with the method described in Example 2. In this example, the His-YoeB protein was added in twice the amount of the h-ΔCwlS, 6His-C-CwlO, or 6His-C-YddH protein by mole. The results of assay of the cell wall lysing activity of the CwlS protein are shown in Fig. 4, the results of assay of the cell wall lysing activity of the CwlO protein are shown in Fig. 5, and the results of assay of the cell wall lysing activity of the YddH protein are shown in Fig. 6.

As shown in Figs. 4 to 6, the YoeB protein was found to be capable of inhibiting the cell wall lysing activity of lytic enzymes, such as CwlS, CwlO, and YddH proteins. By allowing the YoeB protein to act in twice the amount of such enzyme by mole, substantially 100% of the cell wall lysing activity of lytic enzymes was found to be inhibited.

### [Example 4] Effects of YoeB protein for inhibiting lytic enzyme activity 3

In this example, effects of lysis inhibition when YoeB proteins were overexpressed in the Bacillus subtilis mutant Dpr8 lacking 8 types of secretory proteases (Δepr/ΔwprA/Δmpr/ΔnprB/Δbpr/ΔnprE/Δvpr/ΔaprE) were examined. The Dpr8 strain lacks 8 types of known secretory proteases and thus is more likely than a wild-type strain to undergo lysis.

The Dpr8 strain was constructed in the following manner. A list of primers used for constructing the Dpr8 strain is shown in Table 2.

**Table 2**

| Primer | Nucleotide sequence | SEQ ID NO: |
|---|---|---|
| eprfw1 | CTCCGGCAGAAAGGGCAT | 7 |
| eprUPr | CGTTCAGCCGTACAACAAGTTTGCAAGACATG | 8 |
| eprDNf | AACTTGTTGTACGGCTGAACGCCGTCAAACC | 9 |
| eprrv-repU | CTGATCTCGACTTCGTTCAGACGGGTCGTACAATGGCTG | 10 |
| repUfw | GAACGAAGTCGAGATCAG | 11 |
| Cmrv1 | GCTGTAATATAAAAACCTTCT | 12 |
| eprfw2 | CGACACCATAGCTTTCTG | 13 |
| Cmrv2 | AACTAACGGGGCAGGTTA | 14 |
| repUr-CmU | AGAATCAATCCCATGGTCTCACTTTTCCACTTTTTGTCTTG | 15 |
| CmUf-repU | GTGAGACCATGGGATTGATTCTAATGAAGAAAGCAGACAAG | 16 |
| wprAfw 1 | GGGTAATTTATCTGATAGGG | 17 |
| wprAUPr | CTTTTGCTTCCCACAACCGAGCTGAATTTTCTG | 18 |
| wprADNf | CTCGGTTGTGGGAAGCAAAAGTTGTTGTTGAAAA | 19 |
| wprArv-repU | CTGATCTCGACTTCGTTCATCCTCATTGAAGACGGCATC | 20 |
| wprAfw2 | GGAACATATATGACACACCT | 21 |
| mprfw 1 | TGTTTGGTGTTGAGCTGTT | 22 |
| mprUPr | TTCGTGTGAATCCATTGTTTTCTGAATCTTGGAA | 23 |
| mprDNf | GAAAACAATGGATTCACACGAACGGAGGATCGT | 24 |
| mprrv-repU | CTGATCTCGACTTCGTTCCTCGTAAGAAAAAATACCTATTTC | 25 |
| mprfw2 | CCTTGCGAAAGATAGGGTA | 26 |
| nprBfw1 | TTTTTAGCAGTGGTGCTC | 27 |
| nprBUPr | CATACGCCTTCAGTAATAGAGATGTCTTGGTC | 28 |
| nprBDNf | CTCTATTACTGAAGGCGTATGAGGCTGTCGGC | 29 |
| nprBrv-repU | CTGATCTCGACTTCGTTCTTCCAAATGCGCTTCATTAGGA | 30 |
| nprBfw2 | CTTTTGAGCCCGTTCCTC | 31 |
| bprfw 1 | TGAGATTGTGGTGACAGTG | 32 |
| bprUPr | TTAAGTTTTCCGCTGATGAGTCTGTTTTTCGTT | 33 |
| bprDNf | GACTCATCAGCGGAAAACTTAATATGAACACAGAA | 34 |
| bprrv-repU | CTGATCTCGACTTCGTTCGTAATCATGACACCGTTTTGAAC | 35 |
| bprfw2 | ATTGCAACCGGCTTTATCG | 36 |
| nprEfw1 | TTTTGAAGACGTTCGGCGA | 37 |
| nprEUPr | ATTGTCTGTTGCTGAAGCAGCCTGGAATGCTGTT | 38 |
| nprEDNf | CAGGCTGCTTCAGCAACAGACAATTTCTTACCTA | 39 |
| nprErv-repU | CTGATCTCGACTTCGTTCCCTCTCTTAAGTAAGCGCTG | 40 |
| nprEfw2 | TCTCTTTGTTGAAAAACGATA | 41 |
| vprfw 1 | AAAAACATCCCTCCGCTTC | 42 |
| vprUPr | TCTTCGGTCAATACAAGCAGAAAGCGAATGAT | 43 |
| vprDNf | TCTGCTTGTATTGACCGAAGAACCTTTCACTG | 44 |
| vprrv-repU | CTGATCTCGACTTCGTTCTGCTCGGCTCATCTGGAGAAA | 45 |
| vprfw2 | TTTTTGGCAGGCAGCCTT | 46 |
| aprEfw1 | CTGTTTATTATGGGCCACGAA | 47 |
| aprEUPr | GATCAGCTTGGGGTTAATCAACGTACAAGCAG | 48 |
| aprEDNf | TGATTAACCCCAAGCTGATCCACAATTTTTTGC | 49 |
| aprErv-repu | CTGATCTCGACTTCGTTCTGATTTTCCAAACGAGCTTTC | 50 |
| aprEfw2 | ATGGGCCATTATGTCATGAAG | 51 |

Primers used for the deletion of the genes are summarized in Table 3.

**Table 3**

| For deletion of epr genre | For deletion of wprA gene | For deletion of mpr gene | For deletion of nprB gene | For deletion of bpr gene | For deletion of nprE gene | For deletion of vpr gene | For deletion of aprE gene |
|---|---|---|---|---|---|---|---|
| eprfw1 | wprAfw1 | mprfw1 | nprBfw1 | bprfw 1 | nprEfw1 | vprfw1 | aprEfw1 |
| eprUPr | wprAUPr | mprUPr | nprBUPr | bprUPr | nprEUPr | vprUPr | aprEUPr |
| eprDNf | wprADNf | mprDNf | nprBDNf | bprDNf | nprEDNf | vprDNf | aprEDNf |
| eprrv-repU | wprArv-repU | mprrv-repU | nprBrv-repU | bprrv-repU | nprErv-repU | vprrv-repU | aprErv-repu |
| eprfw2 | wprAfw2 | mprfw2 | nprBfww2 | bprfw2 | nprEfw2 | vprfw2 | aprEfw2 |
| Cmrv2 | Cmrv2 | Cmrv2 | Cmrv2 | Cmrv2 | Cmrv2 | Cmrv2 | Cmrv2 |
| repUfw | repUfw | repUfw | repUfw | repUfw | repUfw | repUfw | repUfw |
| Cmrv1 | Cmrv1 | Cmrv1 | Cmrv1 | Cmrv1 | Cmrv1 | Cmrv1 | Cmrv1 |
| repUr-CmU | repUr-CmU | repUr-CmU | repUr-CmU | repUr-Cmu | repUr-Cmu | repUr-Cmu | repUr-CmU |
| CmUf-repU | CmUf-repU | CmUf-repU | CmUf-repU | CmUf-repU | CmUf-repU | CmUf-repU | CmUf-repU |

A method for deleting the epr gene from the wild-type Bacillus subtilis 168 strain is first described. Genomic DNA extracted from the Bacillus subtilis 168 strain was used as a template, a primer set of eprfw1 and eprUpr and a primer set of eprDNf and eprrv-rep shown in Table 2 were used, and a 0.6-kb fragment (A) adjacent to a region upstream of the epr gene and a 0.5-kb fragment (B) adjacent to a region downstream of the epr gene of the genome were prepared. Separately, a promoter region of the repU gene (Nucleic Acids Res., 17, 4410, 1989) derived from the pUB110 plasmid (Plasmid 15, 93, 1986) was ligated to a upstream region of the chloramphenicol resistance gene derived from the pC194 plasmid (J. Bacteriol. 150 (2), 815, 1982) to prepare a 1.2-kb fragment (C). Subsequently, the 3 obtained fragments (A), (B), and (C) were mixed to prepare a template, and SOE-PCR was carried out using the eprfw2 and Cmrv2 primers shown in Table 2 to ligate the 3 fragments in the order of (A), (B), and (C) to obtain a 2.2-kb DNA fragment. The terminuses of the DNA fragment were blunt-ended and 5'-phosphorylated, and the resultant was inserted into the Smal restriction enzyme site of the pUC118 plasmid (Methods Enzymol. 153, 3, 1987) to construct the pUC118-CmrΔepr plasmid for deleting the epr gene. The aforementioned 1.2-kb fragment (C) was prepared via SOE-PCR using a mixture of a 0.4-kb fragment (D) and a 0.8-kb fragment (E) as a template and the repUfw and Cmrv1 primers shown in Table 2. Specifically, the 0.4-kb fragment (D) contains the repU gene promoter region prepared with the use of the primer set of repUfw and repUr-Cm (Table 2) and the pUB110 plasmid as a template, and the 0.8-kb fragment (E) contains the chloramphenicol resistance gene prepared with the use of the primer set of CmUf-rep and Cmrv1 and the pC194 plasmid as a template.

Subsequently, the pUC118-CmrΔepr plasmid for deleting the epr gene prepared as described above was introduced into the Bacillus subtilis 168 strain by the competent cell transformation technique (J. Bacteriol. 93, 1925, 1967), and a transformed strain fused with genomic DNA via single-cross-over homologous recombination between corresponding upstream or downstream regions of the epr gene was obtained using chloramphenicol resistance as an indicator. The resulting transformed strain was inoculated in LB medium, cultured at 37°C for 2 hours, and then subjected to competence induction again. Thus, homologous intergenomic recombination was induced between the overlapping upstream or downstream regions of the genome of the epr gene. When homologous intergenomic recombination takes place in a region different from the region at the time of plasmid introduction, the epr gene would be deleted along with dropout of the plasmid-derived chloramphenicol resistance gene and the pUC118 vector region. Subsequently, ampicillin concentration was carried out in the following manner, in order to enhance the abundance of chloramphenicol-sensitive strains. The culture solution after the competent cells were induced was inoculated in 1 ml of LB medium containing chloramphenicol (final concentration: 5 ppm) and ampicillin sodium (final concentration: 100 ppm) to the turbidity at 600 nm (OD₆₀₀) of 0.003. After culture was conducted at 37°C for 5 hours, 10 µl of an aqueous solution of 10,000 ppm ampicillin sodium was added, and culture was conducted for an additional 3 hours. After the completion of culture, strains were washed via centrifugation with the use of an aqueous solution of 2% sodium chloride, the resultant was suspended in 1 ml of an aqueous solution of 2% sodium chloride, and LB agar medium was coated with 100 µl of the suspension. The strains were incubated at 37°C for approximately 15 hours, and the strains that had become sensitive to chloramphenicol because of dropout of the plasmid region were selected from among the grown strains. The genomic DNA of the selected strains was used as a template to perform PCR using the eprfw2 and eprrv-rep primers shown in Table 2, thereby confirming deletion of the epr gene. Thus, the epr gene-deficient strain was obtained.

Subsequently, the resulting epr gene-deficient strain was subjected to the next deletion; i.e., deletion of the wprA gene, in the same manner as with the deletion of the epr gene. Specifically, the pUC118-CmrΔwprA plasmid for deletion of the wprA gene was constructed in the same manner, the resulting plasmid was introduced into genomic DNA, and the wprA gene was deleted via subsequent homologous intergenomic recombination to obtain a strain lacking both the epr and wprA genes. Subsequently, the same procedure was repeated to successively delete mpr, nprB, bpr, nprE, vpr, and aprE genes, a 8-protease-deficient strain lacking 8 types of protease genes was constructed at last, and the strain was designated as the Dpr8 strain.

The pDG148YB plasmid for overexpressing yoeB to be introduced into the Dpr8 strain was prepared in the following manner. At the outset, the yoeBSal-F-2 (5'-GCGCAGATCTGTCGACTAAGAGAAAGAGATTTTGAAGG (SEQ ID NO: 52)) and yoeBSph-R (5'-GCGCGCATGCTTATATAACTGCGTCAAATTG (SEQ ID NO: 53)) primers for amplifying the yoeB gene were used to perform PCR amplification using the chromosome DNA of the wild-type Bacillus subtilis strain as a template. The amplified fragment (595 bp) was digested with SalI and SphI restriction enzymes. The fragment was ligated to the pUC118 plasmid, which had also been digested with SalI and SphI restriction enzymes, to obtain pU8YB. After the nucleotide sequence of the insert was confirmed, pU8YB was digested with SalI and SphI restriction enzymes. The cleaved yoeB gene fragment (575 bp) was ligated to pDG148, which had also been digested with SalI and SphI restriction enzymes, to obtain pDG148YB of interest. pDG148 is a multicopy plasmid having a promoter that is induced to express by IPTG and it is available from the Bacillus Genetic Stock Center.

The pDG148YB prepared as above was introduced into the Dpr8 strain, and the following experiment was carried out. Pre-culture was conducted in LB medium at 30°C for 15 hours: A pre-culture solution (600 µl) was added to 30 ml of 2×L medium containing 7.5% maltose, 7.5 µg/ml MnSO₄, and 10 µg/ml kanamycin, and main culture was then initiated. In order to induce overexpression of yoeB, IPTG was added to the main culture solution 0, 8, and 20 hours after the initiation of culture to a final concentration of 1 mM in each case. A sample to which IPTG was not added was designated as a control sample. The turbidity of the culture solution at OD₆₀₀ was assayed with the elapse of time, and the effects of lysis inhibition were examined.

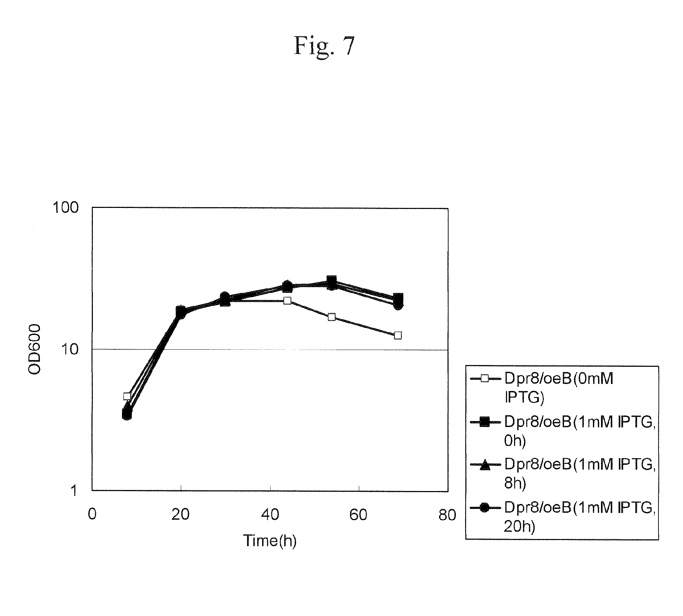

The results are shown in Fig. 7. As is apparent from Fig. 7, the sample to which IPTG was not added began to exhibit lowered turbidity about 30 hours after the initiation of culture. That is, lysis of the Dpr8 strain was observed. In the samples in which YoeB proteins had been overexpressed with the addition of IPTG, however, lowered turbidity was not observed 30 hours after the initiation of culture. This demonstrates that lysis was inhibited in the Dpr8 strain in which YoeB proteins had been overexpressed.

### [Example 5] Activity of YoeB protein for inhibiting PGA degradation

In this example, the effects of the YoeB protein (SEQ ID NO: 2) encoded by the YoeB gene (SEQ ID NO: 1) for inhibiting PGA degradation were examined. Specifically, various types of lytic enzymes were allowed to act on PGAs of a given range of molecular weights, and degradation of PGAs (i.e., reduction of molecular weight) was compared in the presence of or in the absence of the YoeB protein. The various types of lytic enzymes and the YoeB protein used in this example were those used in Examples 2 and 3.

In this example, PGA products (No. 168-21413: Wako Pure Chemical Industries, Ltd.) having average molecular weights of 4,000 kDa to 6,000 kDa were used. The degradation reaction of PGA was carried out using 1 nmol of lytic enzyme and 2 mg of PGA in 10 mM sodium phosphate buffer (pH 7.0). When the YoeB protein was to be added, the amount thereof was 2 nmol. The reaction temperature was 37°C. After a given reaction time had elapsed, the reaction was terminated via boiling at 100°C for 5 minutes. After the termination of the reaction, the sample was subjected to electrophoresis on 14% SDS-polyacrylamide gel, and PGA degradation was confirmed via methylene blue staining.

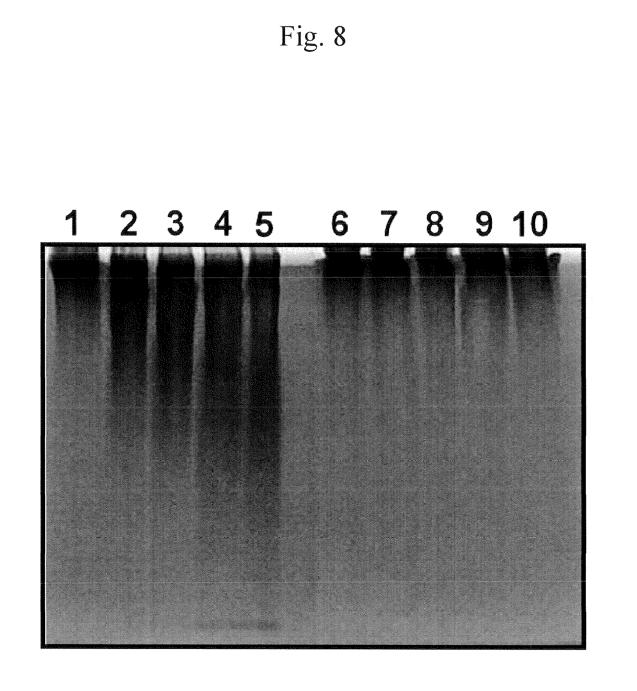

Specifically, the reaction solution having the composition shown in Table 4 was prepared for the CwlE protein. The results of examining the PGA degrading activity of the CwlE protein are shown in Fig. 8.

**Table 4**

| Sample No. | 1 | 2 | 3 |
|---|---|---|---|
| PGA | 100 µl (2 mg) | 100 µl (2 mg) | 100 µl (2 mg) |
| His-CwlE | - | 23 µl (1 nmol) | 23 µl (1 nmol) |
| His-YoeB | - | - | 96 µl (2 nmol) |
| Buffer | 300 µl | 277 µl | 181 µl |
| Total | 400 µl | 400 µl | 400 µl |

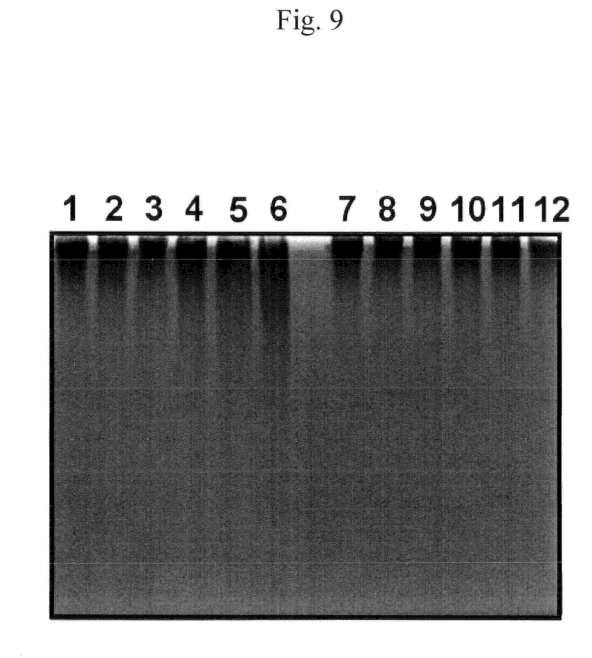

The reaction solution having the composition shown in Table 5 was prepared for the CwlF protein. The results of examining the PGA degrading activity of the CwlF protein are shown in Fig. 9.

**Table 5**

| Sample No. | 1 | 2 | 3 |
|---|---|---|---|
| PGA | 100 µl (2 mg) | 100 µl (2 mg) | 100 µl (2 mg) |
| His-CwlF | - | 63 µl (1 nmol) | 63 µl (1 nmol) |
| His-YoeB | - | - | 146 µl (2 nmol) |
| Buffer | 300 µl | 237 µl | 91 µl |
| Total | 400 µl | 400 µl | 400 µl |

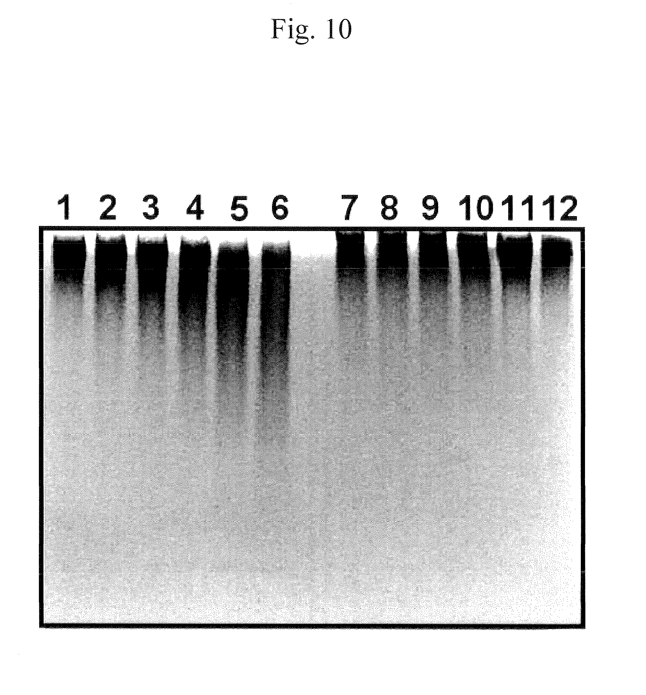

The reaction solution having the composition shown in Table 6 was prepared for the CwlS protein. The results of examining the PGA degrading activity of the CwlS protein are shown in Fig. 10.

**Table 6**

| Sample No. | 1 | 2 | 3 |
|---|---|---|---|
| PGA | 100 µl (2 mg) | 100 µl (2 mg) | 100 µl (2 mg) |
| His-CwlS | - | 40 µl (1 nmol) | 40 µl (1 nmol) |
| His-YoeB | - | - | 146 µl (2 nmol) |
| Buffer | 300 µl | 260 µl | 114 µl |
| Total | 400 µl | 400 µl | 400 µl |

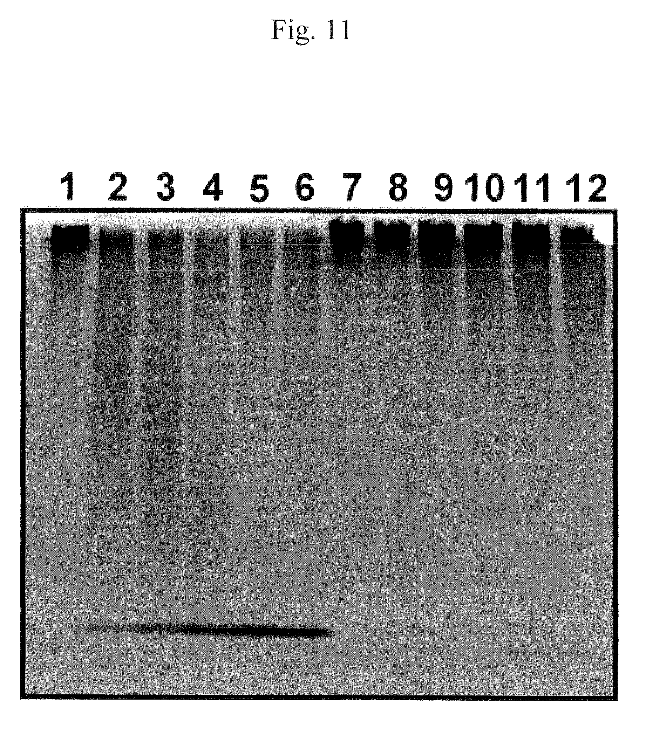

The reaction solution having the composition shown in Table 7 was prepared for the CwlO protein. The results of examining the PGA degrading activity of the CwlO protein are shown in Fig. 11.

**Table 7**

| Sample No. | 1 | 2 | 3 |
|---|---|---|---|
| PGA | 100 µl (2 mg) | 100 µl (2 mg) | 100 µl (2 mg) |
| His-CwlO | - | 13 µl (1 nmol) | 13 µl (1 nmol) |
| His-YoeB | - | - | 146 µl (2 nmol) |
| Buffer | 300 µl | 287 µl | 141 µl |
| Total | 400 µl | 400 µl | 400 µl |

In Fig. 9, lane 1 represents a reaction solution of sample No. 1, lanes 2 to 5 represent the reaction solution of sample No. 2 that has been subjected to the reaction for 0.5 hours, 1.0 hour, 2.0 hours, and 4.0 hours, respectively, and lanes 6 to 10 represent the reaction solution of sample No. 3 that has been subjected to the reaction for 0 hours, 0.5 hours, 1.0 hour, 2.0 hours, and 4.0 hours, respectively. In Figs. 10 and 11, lane 1 represents a reaction solution of sample No. 1, lanes 2 to 6 represent the reaction solution of sample No. 2 that has been subjected to the reaction for 0.5 hours, 1.0 hour, 2.0 hours, 4.0 hours, and 6.0 hours, respectively, and lanes 7 to 12 represent the reaction solution of sample No. 3 that has been subjected to the reaction for 0 hours, 0.5 hours, 1.0 hour, 2.0 hours, 4.0 hours, and 6.0 hours, respectively.

As is apparent from Figs. 9 to 11, the CwlE, CwlF, CwlS, and CwlO proteins have activity of completely degrading PGA and reducing the molecular weight thereof in the absence of the YoeB protein. Also, activity of PGA degradation of the CwlE, CwlF, CwlS, and CwlO proteins was found to be inhibited in the presence of the YoeB protein, and PGA was found to maintain the given range of molecular weights without being degraded. This example demonstrated that the YoeB protein has a novel effect of inhibiting PGA degrading activity of the lytic enzyme.

For the purpose of comparison, whether or not the YwtD protein, which is known to have no cell wall lysing activity but to have PGA degrading activity, would exhibit the effects of inhibiting PGA degradation by the YoeB protein was examined. The nucleotide sequence of the YwtD gene is shown in SEQ ID NO: 66, and the amino acid sequence of the YwtD protein is shown in SEQ ID NO: 67.

The YwtD protein was obtained in the same manner as in the case of <Preparation of C-terminal active domain of CwlS> of Example 3. Specifically, the DNA sequence of the YwtD gene containing the D,L-endopeptidase domain was amplified via PCR using the YwtD-FL-Fw primer (gcgcggtaccgatacatcatcagaattgatt: SEQ ID NO: 68) and the YwtD-FL-Rv primer (gcgcctgcagttattgcacccgtatacttc: SEQ ID NO: 69) and the genomic DNA extracted from the Bacillus subtilis 168 strain as a template. The underlined nucleotide sequences of the above YwtD-FL-Fw and YwtD-FL-Rv primers indicate the KpnI and PstI restriction enzyme recognition sequences, respectively.

The resulting PCR product was used to prepare the pQEYwtD plasmid in the same manner as with the above <Preparation of C-terminal active domain of CwlS>, and E. coli M13 (pREP4, pQEYwtD) into which pQEYwtD had been introduced was obtained. The resulting M13 (pREP4, pQEYwtD) was used to prepare the h-YwtD protein solution in the same manner as with the above <Preparation of C-terminal active domain of CwlS>. The PGA degradation reaction using the resulting YwtD protein was carried out by preparing a reaction solution having the composition shown in Table 8 in the same manner as described above.

**Table 8**

| Sample No. | 1 | 2 | 3 |
|---|---|---|---|
| PGA | 100 µl (2 mg) | 100 µl (2 mg) | 100 µl (2 mg) |
| His-YwtD | - | 77 µl (1 nmol) | 77 µl (1 nmol) |
| His-YoeB | - | - | 96 µl (2 nmol) |
| Buffer | 300 µl | 223 µl | 127 µl |
| Total | 400 µl | 400 µl | 400 µl |

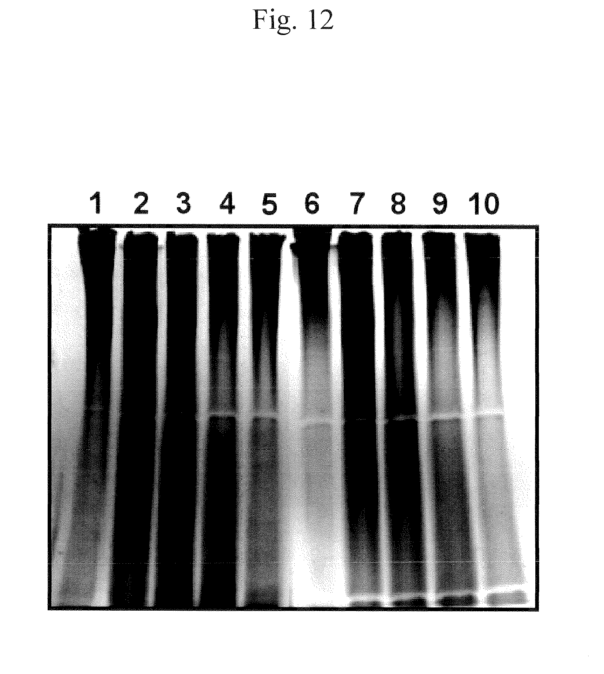

The results are shown in Fig. 12. In Fig. 12, lane 1 represents a reaction solution of sample No. 1, lanes 2 to 5 represent the reaction solution of sample No. 2 that has been subjected to the reaction for 0.5 hours, 1.0 hour, 2.0 hours, and 4.0 hours, respectively, and lanes 6 to 10 represent the reaction solution of sample No. 3 that has been subjected to the reaction for 0 hours, 0.5 hours, 1.0 hour, 2.0 hours, and 4.0 hours, respectively. As is apparent from Fig. 12, PGA degrading activity of the YwtD protein was not different in the presence of and in the absence of the YoeB protein. Specifically, the YoeB protein was found not to inhibit PGA degrading activity of the YwtD protein.

It was thus demonstrated that the YoeB protein would inhibit PGA degrading activity of a lytic enzyme having cell wall lysing activity but would not inhibit PGA degrading activity of a PGA degrading enzyme having no cell wall lysing activity.

### Industrial Applicability

The present invention can provide a novel lytic enzyme inhibitor that can effectively inhibit, for example, activity of a Bacillus subtilis-derived lytic enzyme and a lysis inhibitor that can inhibit, for example, lysis of Bacillus subtilis. Specifically, the lytic enzyme inhibitor of the present invention can inhibit lytic activity of various types of lytic enzymes, including Bacillus subtilis-derived lytic enzymes. Also, the lysis inhibitor of the present invention can inhibit lysis of various types of microorganisms, including Bacillus subtilis.

Also, the present invention can provide a microorganism exhibiting inhibition of lysis by a lytic enzyme. With the use of the microorganism of the present invention as, for example, a host for material production, the productivity of such material can be improved because lysis thereof has been inhibited.

Further, the present invention can provide an inhibitor of PGA degradation that inhibits degradation of PGA caused by a lytic enzyme. Also, the present invention can provide a method for producing a PGA of a higher molecular weight than a wild-type strain.

All publications, patents, and patent applications cited herein are incorporated herein by reference in their entirety.

## Claims

1. A lytic enzyme inhibitor composed mainly of the Bacillus subtilis-derived YoeB gene or the YoeB protein encoded by a gene homologous to the YoeB gene.

2. The lytic enzyme inhibitor according to claim 1, wherein the YoeB protein is any of proteins (a) to (c) below:
(a) a protein consisting of the amino acid sequence as shown in SEQ ID NO: 2;
(b) a protein consisting of an amino acid sequence derived from the amino acid sequence as shown in SEQ ID NO: 2 by substitution, deletion, addition, or insertion of one or several amino acids and having activity of binding to the Bacillus subtilis-derived lytic enzyme to inhibit lytic activity thereof; and
(c) a protein consisting of an amino acid sequence having 70% or higher homology to the amino acid sequence as shown in SEQ ID NO: 2 and having activity of binding to the Bacillus subtilis-derived lytic enzyme to inhibit lytic activity thereof.

3. The lytic enzyme inhibitor according to claim 1 or 2, which inhibits lytic activity of at least 1 type of lytic enzyme selected from the group consisting of the Bacillus subtilis-derived CwlE, CwlF, CwlS, CwlO, and YddH proteins and proteins functionally equivalent thereto.

4. The lytic enzyme inhibitor according to claim 3, wherein the CwlE protein is any of the proteins (a) to (c) below:
(a) a protein consisting of the amino acid sequence as shown in SEQ ID NO: 4;
(b) a protein consisting of an amino acid sequence derived from the amino acid sequence as shown in SEQ ID NO: 4 by substitution, deletion, addition, or insertion of one or several amino acids and having activity of lysing the cell wall of Bacillus subtilis; and
(c) a protein consisting of an amino acid sequence having 70% or higher homology to the amino acid sequence as shown in SEQ ID NO: 4 and having activity of lysing the cell wall of Bacillus subtilis.

5. The lytic enzyme inhibitor according to claim 3, wherein the CwlF protein is any of proteins (a) to (c) below:
(a) a protein consisting of the amino acid sequence as shown in SEQ ID NO: 6;
(b) a protein consisting of an amino acid sequence derived from the amino acid sequence as shown in SEQ ID NO: 6 by substitution, deletion, addition, or insertion of one or several amino acids and having activity of lysing the cell wall of Bacillus subtilis; and
(c) a protein consisting of an amino acid sequence having 70% or higher homology to the amino acid sequence as shown in SEQ ID NO: 6 and having activity of lysing the cell wall of Bacillus subtilis.

6. The lytic enzyme inhibitor according to claim 3, wherein the CwlS protein is any of the proteins (a) to (c) below:
(a) a protein consisting of the amino acid sequence as shown in SEQ ID NO: 55;
(b) a protein consisting of an amino acid sequence derived from the amino acid sequence as shown in SEQ ID NO: 55 by substitution, deletion, addition, or insertion of one or several amino acids and having activity of lysing the cell wall of Bacillus subtilis; and
(c) a protein consisting of an amino acid sequence having 70% or higher homology to the amino acid sequence as shown in SEQ ID NO: 55 and having activity of lysing the cell wall of Bacillus subtilis.

7. The lytic enzyme inhibitor according to claim 3, wherein the CwlO protein is any of the proteins (a) to (c) below;
(a) a protein consisting of the amino acid sequence as shown in SEQ ID NO: 57;
(b) a protein consisting of an amino acid sequence derived from the amino acid sequence as shown in SEQ ID NO: 57 by substitution, deletion, addition, or insertion of one or several amino acids and having activity of lysing the cell wall of Bacillus subtilis; and
(c) a protein consisting of an amino acid sequence having 70% or higher homology to the amino acid sequence as shown in SEQ ID NO: 57 and having activity of lysing the cell wall of Bacillus subtilis.

8. The lytic enzyme inhibitor according to claim 3, wherein the YddH protein is any of the proteins (a) to (c) below:
(a) a protein consisting of the amino acid sequence as shown in SEQ ID NO: 59;
(b) a protein consisting of an amino acid sequence derived from the amino acid sequence as shown in SEQ ID NO: 59 by substitution, deletion, addition, or insertion of one or several amino acids and having activity of lysing the cell wall of Bacillus subtilis; and
(c) a protein consisting of an amino acid sequence having 70% or higher homology to the amino acid sequence as shown in SEQ ID NO: 59 and having activity of lysing the cell wall of Bacillus subtilis.

9. A lysis inhibitor composed mainly of the Bacillus subtilis-derived YoeB gene or the YoeB protein encoded by a gene homologous to the YoeB gene.

10. The lysis inhibitor according to claim 9, wherein the YoeB protein is any of the proteins (a) to (c) below:
(a) a protein consisting of the amino acid sequence as shown in SEQ ID NO: 2;
(b) a protein consisting of an amino acid sequence derived from the amino acid sequence as shown in SEQ ID NO: 2 by substitution, deletion, addition, or insertion of one or several amino acids and having activity of binding to the Bacillus subtilis-derived lytic enzyme to inhibit lytic activity thereof; and
(c) a protein consisting of an amino acid sequence having 70% or higher homology to the amino acid sequence as shown in SEQ ID NO: 2 and having activity of binding to the Bacillus subtilis-derived lytic enzyme to inhibit lytic activity thereof.

11. The lysis inhibitor according to claim 9 or 10, which inhibits lysis of Bacillus subtilis.

12. The lysis inhibitor according to claim 11, wherein the Bacillus subtilis is a mutant that lacks at least one type of secretory protease.

13. An inhibitor of poly-γ-glutamic acid degradation composed mainly of the Bacillus subtilis-derived YoeB gene or the YoeB protein encoded by a gene homologous to the YoeB gene.

14. The inhibitor of poly-γ-glutamic acid degradation according to claim 13, wherein the YoeB protein is any of the proteins (a) to (c) below:
(a) a protein consisting of the amino acid sequence as shown in SEQ ID NO: 2;
(b) a protein consisting of an amino acid sequence derived from the amino acid sequence as shown in SEQ ID NO: 2 by substitution, deletion, addition, or insertion of one or several amino acids and having activity of binding to the Bacillus subtilis-derived lytic enzyme to inhibit lytic activity thereof; and
(c) a protein consisting of an amino acid sequence having 70% or higher homology to the amino acid sequence as shown in SEQ ID NO: 2 and having activity of binding to the Bacillus subtilis-derived lytic enzyme to inhibit lytic activity thereof.

15. The inhibitor of poly-γ-glutamic acid degradation according to claim 13 or 14, wherein the YoeB protein inhibits lytic activity of at least 1 lytic enzyme selected from the group consisting of the Bacillus subtilis-derived CwlE, CwlF, CwlS, and CwlO proteins and proteins functionally equivalent to said proteins to inhibit degradation of poly-γ-glutamic acid.

16. The inhibitor of poly-γ-glutamic acid degradation according to claim 15, wherein the CwlE protein is any of proteins (a) to (c) below:
(a) a protein consisting of the amino acid sequence as shown in SEQ ID NO: 4;
(b) a protein consisting of an amino acid sequence derived from the amino acid sequence as shown in SEQ ID NO: 4 by substitution, deletion, addition, or insertion of one or several amino acids and having activity of lysing the cell wall of Bacillus subtilis; and
(c) a protein consisting of an amino acid sequence having 70% or higher homology to the amino acid sequence as shown in SEQ ID NO: 4 and having activity of lysing the cell wall of Bacillus subtilis.

17. The inhibitor of poly-γ-glutamic acid degradation according to claim 15, wherein the CwlF protein is any of proteins (a) to (c) below:
(a) a protein consisting of the amino acid sequence as shown in SEQ ID NO: 6;
(b) a protein consisting of an amino acid sequence derived from the amino acid sequence as shown in SEQ ID NO: 6 by substitution, deletion, addition, or insertion of one or several amino acids and having activity of lysing the cell wall of Bacillus subtilis; and
(c) a protein consisting of an amino acid sequence having 70% or higher homology to the amino acid sequence as shown in SEQ ID NO: 6 and having activity of lysing the cell wall of Bacillus subtilis.

18. The inhibitor of poly-γ-glutamic acid degradation according to claim 15, wherein the CwlS protein is any of proteins (a) to (c) below:
(a) a protein consisting of the amino acid sequence as shown in SEQ ID NO: 55;
(b) a protein consisting of an amino acid sequence derived from the amino acid sequence as shown in SEQ ID NO: 55 by substitution, deletion, addition, or insertion of one or several amino acids and having activity of lysing the cell wall of Bacillus subtilis; and
(c) a protein consisting of an amino acid sequence having 70% or higher homology to the amino acid sequence as shown in SEQ ID NO: 55 and having activity of lysing the cell wall of Bacillus subtilis.

19. The inhibitor of poly-γ-glutamic acid degradation according to claim 15, wherein the CwlO protein is any of proteins (a) to (c) below:
(a) a protein consisting of the amino acid sequence as shown in SEQ ID NO: 57;
(b) a protein consisting of an amino acid sequence derived from the amino acid sequence as shown in SEQ ID NO: 57 by substitution, deletion, addition, or insertion of one or several amino acids and having activity of lysing the cell wall of Bacillus subtilis; and
(c) a protein consisting of an amino acid sequence having 70% or higher homology to the amino acid sequence as shown in SEQ ID NO: 57 and having activity of lysing the cell wall of Bacillus subtilis.

20. A microorganism whose lysis is inhibited by expressing the Bacillus subtilis-derived YoeB gene or a gene homologous to the YoeB gene under the control of an expression-inducible promoter.

21. The microorganism according to claim 20, wherein the YoeB gene encodes any of proteins (a) to (c) below:
(a) a protein consisting of the amino acid sequence as shown in SEQ ID NO: 2;
(b) a protein consisting of an amino acid sequence derived from the amino acid sequence as shown in SEQ ID NO: 2 by substitution, deletion, addition, or insertion of one or several amino acids and having activity of binding to the Bacillus subtilis-derived lytic enzyme to inhibit lytic activity thereof; and
(c) a protein consisting of an amino acid sequence having 70% or higher homology to the amino acid sequence as shown in SEQ ID NO: 2 and having activity of binding to the Bacillus subtilis-derived lytic enzyme to inhibit lytic activity thereof.

22. The microorganism according to claim 20 or 21, which lacks at least 1 secretory protease.

23. The microorganism according to any 1 of claims 20 to 22, which is a Bacillus subtilis-derived strain.

24. The microorganism according to any 1 of claims 20 to 23, which is capable of producing poly-γ-glutamic acid.

25. The microorganism according to claim 24, wherein the YoeB gene or the YoeB protein encoded by a gene homologous to the YoeB gene inhibits lytic activity of at least 1 lytic enzyme selected from the group consisting of the Bacillus subtilis-derived CwlE, CwlF, CwlS, and CwlO proteins and proteins functionally equivalent to said proteins to inhibit reduction of the molecular weight of poly-γ-glutamic acid.

26. The microorganism according to claim 25, wherein the CwlE protein is any of proteins (a) to (c) below:
(a) a protein consisting of the amino acid sequence as shown in SEQ ID NO: 4;
(b) a protein consisting of an amino acid sequence derived from the amino acid sequence as shown in SEQ ID NO: 4 by substitution, deletion, addition, or insertion of one or several amino acids and having activity of lysing the cell wall of Bacillus subtilis; and
(c) a protein consisting of an amino acid sequence having 70% or higher homology to the amino acid sequence as shown in SEQ ID NO: 4 and having activity of lysing the cell wall of Bacillus subtilis.

27. The microorganism according to claim 25, wherein the CwlF protein is any of proteins (a) to (c) below:
(a) a protein consisting of the amino acid sequence as shown in SEQ ID NO: 6;
(b) a protein consisting of an amino acid sequence derived from the amino acid sequence as shown in SEQ ID NO: 6 by substitution, deletion, addition, or insertion of one or several amino acids and having activity of lysing the cell wall of Bacillus subtilis; and
(c) a protein consisting of an amino acid sequence having 70% or higher homology to the amino acid sequence as shown in SEQ ID NO: 6 and having activity of lysing the cell wall of Bacillus subtilis.

28. The microorganism according to claim 25, wherein the CwlS protein is any of proteins (a) to (c) below:
(a) a protein consisting of the amino acid sequence as shown in SEQ ID NO: 55;
(b) a protein consisting of an amino acid sequence derived from the amino acid sequence as shown in SEQ ID NO: 55 by substitution, deletion, addition, or insertion of one or several amino acids and having activity of lysing the cell wall of Bacillus subtilis; and
(c) a protein consisting of an amino acid sequence having 70% or higher homology to the amino acid sequence as shown in SEQ ID NO: 55 and having activity of lysing the cell wall of Bacillus subtilis.

29. The microorganism according to claim 25, wherein the CwlO protein is any of proteins (a) to (c) below:
(a) a protein consisting of the amino acid sequence as shown in SEQ ID NO: 57;
(b) a protein consisting of an amino acid sequence derived from the amino acid sequence as shown in SEQ ID NO: 57 by substitution, deletion, addition, or insertion of one or several amino acids and having activity of lysing the cell wall of Bacillus subtilis; and
(c) a protein consisting of an amino acid sequence having 70% or higher homology to the amino acid sequence as shown in SEQ ID NO: 57 and having activity of lysing the cell wall of Bacillus subtilis.

30. A method for producing poly-γ-glutamic acid consisting of culturing a microorganism capable of producing poly-γ-glutamic acid in the presence of the Bacillus subtilis-derived YoeB gene or the YoeB protein encoded by a gene homologous to the YoeB gene and recovering the poly-γ-glutamic acid produced in the medium.

31. The method for producing poly-γ-glutamic acid according to claim 30, wherein the poly-γ-glutamic acid has a higher molecular weight than the poly-γ-glutamic acid produced in the absence of the YoeB protein.

32. The method for producing poly-γ-glutamic acid according to claim 30 or 31, wherein the microorganism is the microorganism according to any one of claims 24 to 29.

33. The method for producing poly-γ-glutamic acid according to any one of claims 30 to 32, wherein the YoeB protein is added to the medium.
